# EUROPEAN PATENT SPECIFICATION

(11) **EP 3 746 041 B1**
(45) Date of publication and mention of the grant of the patent: **04.06.2025**
(21) Application number: 19702528.1
(22) Date of filing: 29.01.2019
(51) Int. Cl.: A61K 8/64, A61Q 19/08

(54) **USE OF CYCLIC PEPTIDES IN COSMETIC**
VERWENDUNG VON CYCLISCHEN PEPTIDEN IN DER KOSMETIK
UTILISATION DE PEPTIDES CYCLIQUES DANS UN PRODUIT COSMÉTIQUE

(30) Priority: 01.02.2018 FR 1850845
(43) Date of publication of application: 09.12.2020
(73) Proprietor: SEDERMA, 78610 Le-Perray-en-Yvelines (FR)
(72) Inventor: MONDON, Philippe, 92120 Montrouge (FR); DORIDOT, Emmanuel, 78180 Montigny-le-Bretonneux (FR); MARCHAND, Thibault, 78690 Saint-Remy-l'Honoré (FR)
(74) Representative: de Saint Viance, Isabelle Marie Fanny
(86) International application number: PCT/EP2019/025028
(87) International publication number: WO 2019/149450

(56) References cited:
- WO-A1-2007/032029
- WO-A1-2009/079792
- WO-A1-2013/091070
- US-A1- 2016 030 322

## Description

### TECHNICAL FIELD

The present invention relates to a peptide-based cosmetic treatment, a novel peptide-based cosmetic or dermatological ingredient, compositions comprising it and cosmetic or dermatological uses thereof. More particularly, it relates to peptides intended for the treatment of the skin and its appendages, of human or animal mammals.

It concerns in particular the industries of cosmetic, dermatological products, and the industry of hygiene and personal care products.

### BACKGROUND ART

Peptides have an important signal function and coordinate many biochemical processes. As a result, they have long become unavoidable and promising active ingredients, particularly in the cosmetic industry, where new compounds are constantly being searched that can beautify the skin and the appendages, namely to improve their general condition.

Numerous peptides or peptide mixtures having properties on the dermal extracellular matrix and thus having anti-aging applications have already been proposed, in particular by the Applicant, such as the Pal-KTTKS (SEQ ID NO: 1) sold under the trademark MATRIXYL^{®}, the Pal-GHK/Pal-GQPR mixture (SEQ ID NO: 2) sold under the trademark MATRIXYL^{®} 3000, the Pal-KMO2K sold under the trademark MATRIXYL^{®} Synthe'6^{®} (MO2 corresponding to a dioxygenated methionine) or more recently the Pal-K(P)HG (having a proline grafted on a lysine) sold under the trademark MATRIXYL^{®}Morphomics^{™}. Other known peptides are mentioned below in the description.

In the general class of peptides, there are the cyclic peptides (or cyclopeptides). Many of them have been discovered in nature. They can also be synthesized in laboratories. Their length varies from two amino acid residues to several hundred. They have found many applications in medicine and biology.

The simpliest are peptides having neither -NH₂ terminal group nor terminal COOH group, these two terminal groups having reacted together to form a peptide bond and thus having closed the peptide backbone. There are also some cyclic peptides for which the covalent bond is created between the terminal amine and a side chain or between the terminal carboxyl and a side chain or between two side chains, which makes the peptide molecule partially cyclic.

Cyclic peptides have a number of advantages over conventional peptides. For example, they are insensitive to exo-peptidases which attack the linear peptides by the C and/or N terminal ends to degrade them into amino acids. Cyclic peptides are thus more stable than their linear counterparts. Regarding their activity, due to the curvature of their skeleton and the lesser degree of freedom, many rotations of bonds are blocked, and cyclic peptides have side chains more available for interactions with external targets. The strength of the interactions with their target can thus be increased, the adjacent amino acids not disturbing the molecular interaction. This may give them increased bioavailability over linear peptides.

Plants or microorganisms represent a natural source of cyclic peptides (see, for example, the review of synthesis which lists plant cyclopeptides: "Plant cyclopeptides", Tan, N.H., Zhou, J.; Chem Rev., 2006; 106 (3); 840-895). Therefore, cyclic peptides have also the advantage of being able to be produced by means of an eco-designed chemistry, from a natural material and without solvents dangerous for the environment and health.

In particular, flax seed oil (*Linum usitatissimum L*.) is known to contain cyclic peptides. Flax is part of the Linaceae family. This annual herb is exploited in its entirety for its fiber and oilseeds. Its origin is uncertain. It would substantially come from Eurasia. Domesticated for a very long time (several thousand years ago, first trace 36,000 years ago), flax is now cultivated all over the world. Canada is the largest producer. Linseed oil contains many components. It is rich in triglycerides of unsaturated fatty acids, especially linolenic acid. It also contains mucilage, triterpenes and sterols, cyanogenic heterosides, lignans and proteins (including cyclic peptides). The following medical activities of linseed oil are especially described: laxative, softening on the irritation of the mucous membranes, anti-inflammatory, analgesic and antipyretic, preventive on the cardiovascular system, hormonal, antidiabetic and anticancer.

Some cyclic peptides of linseed oil (*Linum usitatissimum L*.)*,* a by-product of the flax industry, have been proposed in the context of medical applications: against neurodegenerative, hematological, inflammatory and viral infections, autoimmune diseases and cancer (WO200979792), to improve health by stimulating growth, cell cycle and weight gain, by controlling enteritis, fungal growth, modulating microorganisms of the gastrointestinal tract and inhibiting infiltration of inflammatory cells in intestinal mucosa (WO2013091071), against osteoporosis (WO2015/114817) and for immunosuppressive therapy (WO9007523).

In addition, a review summarizes the cyclic peptides of plants used in the Chinese pharmacopoeia ("Chemical Progress in Cyclopeptide-Containing Traditional Medicines Cited in Chinese Pharmacopoeia"); Zhao, Simeng et al; Chin J. Chem., 2012, 30 (6); 1213-1225).

In the field of active ingredients for cosmetics, MERCK has already sold RonaCare^{®} Cyclopeptide-5 as an anti-wrinkle active. This particular cyclic peptide obtained by synthesis (the *Cyclo* (1-aminocyclohexanecarbonyl-L-arginylglycyl-L-alpha-aspartyl-D-phenylalanyl, SEQ ID NO: 3) was the subject of the patent application WO2009/124754. Other cyclic peptides for cosmetic treatment of skin have been disclosed for example in WO2007/032029 and US2016/030322.

### SUMMARY OF THE INVENTION

The aim of the present invention is to provide cyclic peptides which may be provided in the form of an active ingredient, in particular for cosmetics, capable of improving the general condition of the skin and/or its appendages, and more particularly cyclic peptides active not only on the synthesis of the main molecules of the extracellular matrix but also on other complementary biological targets. In addition, the invention aims to provide cyclic peptides sufficiently effective to be used alone or in combination, in proportions of a few ppm, and that can be used in the form of topical composition, including cosmetic composition.

For this purpose, according to a first aspect, the present invention provides the use of at least one cyclic peptide as active component in a non-therapeutic cosmetic treatment of the skin and/or its appendages, said cyclic peptide consisting of at least five amino acids being constituted of at least one proline (Pro) and at least two phenylalanines (Phe), the other amino acids being chosen in the group comprising leucine (Leu), isoleucine (Ile), valine (Val), alanine (Ala), glycine (Gly), methionine (Met) and tryptophan (Trp); the methionine (Met), when present, being non-oxidized or oxidized. Moreover, according to this first aspect, said cyclic peptide comprises at most 15 amino acids, comprises at least one leucine and comprises at least two phenylalanines and/or at least two prolines.

Preferably, the cyclic peptides according to the invention are cyclic peptides formed from a single ring formed solely of a peptide backbone and having neither a terminal -NH₂ group nor a terminal COOH group, as according to the following formula I:

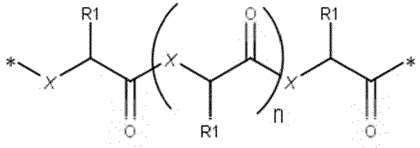

Wherein in formule I:
R1 is a side group of amino acids;
X = NH or, in the case of a proline, X = N and R1 are part of a 5-atom ring;
n = 3 to 13, preferably 3 to 11, more preferably n=6 or n=7 so as to form a cyclic peptide comprising at most 15 amino acids, preferably at most 12, more preferably at most 9 amino acids, preferably comprising 8 or 9 amino acids. Beyond 15, the cyclic peptide may be too expensive to manufacture and/or too bulky to be able to be transported into skin.

As shown by the *in vitro* and *in vivo* results given below in the detailed description, the particular cyclic peptides according to the invention are of interest in the field of active ingredients for the cosmetic industry.

Indeed, they possess the following beneficial activities for the skin and its appendages:
1) a stimulating activity of the synthesis of the two main constituent molecules of the dermal extracellular matrix (ECM) decreasing with age: collagen I and elastin.
   The loss of density and thickness of the dermis, as well as the appearance of wrinkles, are related to a reduction in the synthesis of these macromolecules by dermal fibroblasts during aging. Collagen I is the most abundant protein of the dermis. Collagen I is essential for having a dense and firm skin. Elastin is synthesized and secreted in the extracellular dermal space. It constitutes the major component up to 90% of the elastic fibers. The weakening of the qualities of the dermis by the insufficient renewal of these components also causes the loss of support and protection for the appendages of skin: the blood vessels become more apparent and fragile and the pores become more apparent because of the reduction of their peripheral sheathing. This will negatively affect the homogeneity and texture of the skin.
2) a stimulating activity of the synthesis of two of the main types of the dermal/epidermal junction (DEJ) molecules: collagen IV and all the laminins.

The decrease in synthesis of these molecules leads to poorer communication between melanocytes, keratinocytes and DEJ, and to less flexibility of the system. In contrast, an appropriate synthesis of these molecules helps to restore/strengthen the DEJ. Collagen IV forms a two-dimensional network and is one of the major components of the DEJ. Laminins are also contained in the basal layer and participate in the anchoring of cell surfaces to the basal lamina. Together, these two essential components of the DEJ ensure to the keratinocyte of the basal lamina a better anchoring and help maintaining the flexibility of the epidermis.

The stimulation of the syntheses of these proteins of the dermis and the DEJ makes it possible to obtain results on the embellishment and the general state of the skin, mainly on the level of the mechanical properties: a skin more dense, re-inflated, more firm, more tonic, more flexible and elastic, but also at the visual level with, consequently, a more homogeneous and smooth skin texture.

3) A reduction activity of the production of sebum by the sebocytes in order to reduce the size of the pores and to make them less visible (the skin appears smoother with a more regular grain), and where necessary in order to contribute to decrease the glossy appearance which is characteristic of an oily skin.

4) An activity of lowering the level of mediators of inflammation very present in microinflammatory phenomena which has the effect of reducing sensations of discomfort of sensitive skin and rednesses.

5) A modulation activity of the production of stratifin.

Stratifin is a recently discovered protein synthetised by the epidermis, which is found after in the dermis and acts negatively on its components. The production of this protein by the keratinocyte is increased in oxidizing conditions (UV-type stress for example) that are known to increase premature aging. After migration into the dermis, stratifin causes on the one hand the production by fibroblasts of proteases that attack the dermal proteins (MMP-1 and -2 in particular), and on the other hand, it reduces the production of mRNA collagen type I and fibronectin. Its increase is therefore linked to the degradation of the quality of the supporting tissues and to premature aging. It is therefore interesting to limit the production of stratifin.

6) A stimulating activity of the proteins involved in the proper functioning of the mitochondria.

These proteins participate in the production of energy, the transport of metabolites between the cytoplasm and the mitochondria, and the defense against oxidative stress. The synthesis of these proteins decreases with age (in quantity and quality). Therefore, fighting against skin aging involves the positive regulation of the production of these proteins.

The present invention can thus aim a cosmetic treatment intended to improve the elastic properties, the firmness, the texture and/or the radiance or brightness of the skin. This treatment can be selected from a treatment of wrinkles and fine lines, a smoothing, firming, restructuring, plumping, anti-sagging treatment, to reduce pore size (tightening) and/or to reduce the shine of the skin due to an excess of sebum and/or to reduce cutaneous micro-inflammations.

Other applications are also envisageable for the cyclic peptide(s) according to the invention, especially a moisturizing, slimming, detoxifying, but also anti-glycation, anti-radical, anti-fatigue, anti-undereye bags and/or dark circles, an action on the growth of hairs (head and body), an action on pigmentation, on the scalp, tensor effect etc. as preventive or curative.

According to preferred features, the cyclic peptide comprises particular amino acids and/or particular amino acid sequences, more particularly:
-
- At least one Pro-Phe-Phe sequence; and/or
- At least one Pro-Pro-Phe-Phe sequence; and/or
- One Ile-Met-Leu or Val-Met-Leu sequence; and/or
- Two methionines; and/or
- One tryptophane; and/or
- the Pro-Phe-Phe-Trp sequence.

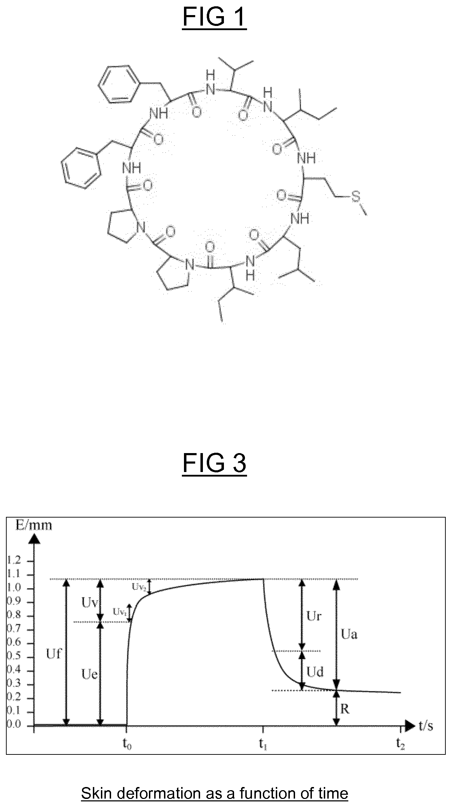

Thus, said at least one cyclic peptide is according to the first aspect of the invention selected from the group comprising:
- The *Cyclo*(-Met-Leu-Val-Phe-Pro-Leu-Phe-Ile) (SEQ ID NO: 4);
- The *Cyclo*(-Met-Leu-Ile-Pro-Pro-Phe-Phe-Val-Ile) (SEQ ID NO: 5; shown at figure 1);
- The *Cyclo*(-Ile-Leu-Val-Pro-Pro-Phe-Phe-Leu-Ile) (SEQ ID NO: 6);
- The *Cyclo*(-Met-Leu-Met-Pro-Phe-Phe-Trp-Ile) (SEQ ID NO: 7);
- The *Cyclo*(-Met-Leu-Leu-Pro-Phe-Phe-Trp-Ile) (SEQ ID NO: 8); and
- The *Cyclo*(-Met-Leu-Met-Pro-Phe-Phe-Trp-Val) (SEQ ID NO: 9).

According to the first aspect of the invention, preferably, as shown by the comparative results given below in the description, a mixture of at least two cyclic peptides is used, said at least two cyclic peptides being chosen from the group comprising the following three cyclic peptides:
- The *Cyclo*(-Met-Leu-Val-Phe-Pro-Leu-Phe-Ile) (SEQ ID NO: 4);
- The *Cyclo*(-Met-Leu-Ile-Pro-Pro-Phe-Phe-Val-Ile) (SEQ ID NO: 5); and
- The *Cyclo*(-Ile-Leu-Val-Pro-Pro-Phe-Phe-Leu-Ile) (SEQ ID NO: 6);
more particularly, a mixture advantageously comprising these three cyclic peptides, in particular a mixture comprising at least 50% by weight, preferably at least 60% by weight, and more preferably at least 70% by weight, with respect to total weight of said mixture of cyclic peptides.

Preferably, the balance in% by weight relative to the total weight of said mixture of cyclic peptides comprises at least one cyclic peptide chosen from the group comprising:
- The *Cyclo*(-Met-Leu-Met-Pro-Phe-Phe-Trp-Ile) (SEQ ID NO: 7);
- The *Cyclo*(-Met-Leu-Leu-Pro-Phe-Phe-Trp-Ile) (SEQ ID NO: 8); and
- The *Cyclo*(-Met-Leu-Met-Pro-Phe-Phe-Trp-Val) (SEQ ID NO: 9).

More particularly, the cyclic peptide mixture comprises:
- Approximately 15 to 25% by weight relative to the total weight of said mixture of cyclic peptides of *Cyclo*(-Met-Leu-Val-Phe-Pro-Leu-Phe-Ile) (SEQ ID NO: 4);
- Approximately 15 to 25% by weight relative to the total weight of said mixture of cyclic peptides of *Cyclo*(-Met-Leu-Ile-Pro-Pro-Phe-Phe-Val-Ile) (SEQ ID NO: 5);
- Approximately 15 to 25% by weight relative to the total weight of said mixture of cyclic peptides of *Cyclo*(-Ile-Leu-Val-Pro-Pro-Phe-Phe-Leu-Ile) (SEQ ID NO: 6); and
- The balance in weight % relative to the total weight of the mixture of cyclic peptides being a mixture of the cyclic peptides *Cyclo*(-Met-Leu-Met-Pro-Phe-Phe-Trp-Ile) (SEQ ID NO: 7), *Cyclo*(-Met-Leu-Leu-Pro-Phe-Phe-Trp-Ile) (SEQ ID NO: 8) and *Cyclo*(-Met-Leu-Met-Pro-Phe-Phe-Trp-Val) (SEQ ID NO: 9).

According to a second aspect, the present invention provides a cosmetic or dermatological active ingredient comprising in a physiologically acceptable medium a mixture of cyclic peptides comprising:
- Approximately 15 to 25% by weight relative to the total weight of said mixture of cyclic peptides of *Cyclo*(-Met-Leu-Val-Phe-Pro-Leu-Phe-Ile) (SEQ ID NO: 4);
- Approximately 15 to 25% by weight relative to the total weight of said mixture of cyclic peptides of *Cyclo*(-Met-Leu-Ile-Pro-Pro-Phe-Phe-Val-Ile) (SEQ ID NO: 5);
- Approximately 15 to 25% by weight relative to the total weight of said mixture of cyclic peptides of *Cyclo*(-Ile-Leu-Val-Pro-Pro-Phe-Phe-Leu-Ile) (SEQ ID NO: 6); and
- The balance in weight % relative to the total weight of the mixture of cyclic peptides being a mixture of the cyclic peptides *Cyclo*(-Met-Leu-Met-Pro-Phe-Phe-Trp-Ile) (SEQ ID NO: 7), *Cyclo*(-Met-Leu-Leu-Pro-Phe-Phe-Trp-Ile) (SEQ ID NO: 8) and *Cyclo*(-Met-Leu-Met-Pro-Phe-Phe-Trp-Val) (SEQ ID NO: 9).

These cyclic peptide(s) may be manufactured by peptide synthesis or may advantageously be extracted from linseed oil (*Linum usitatissimum* L.) with a suitable solvent, in particular an alcoholic solvent, for example vegetable ethanol, or any other solvent with low impact for human and environment, either as a mixture or alone after purification. A preparation method is given below in the detailed description.

Preferably, the cyclic peptides used according to the invention, alone or as a mixture, are partially oxidized, to sulfoxides or sulfone, preferably sulfoxides, and preferably slightly oxidized to sulfoxides, and more preferably being not oxidized.

Commonly, the cyclic peptide(s) may be combined with other active agents, at effective concentrations that can act synergistically or as a reinforcement of activity, such as the following agents: anti-aging, anti-wrinkles and fine lines, lightening, pro-pigmenting, moisturizing, hydrating, astringent, anti-seborrhea, slimming, anti-acne, anti-inflammatory, anti-oxidants, acting on the radiance of the complexion, anti-glycation, volumizers, restructuring, anti-carbonylation, dermo-relaxants, anti-hair regrowth, acting on the *stratum corneum,* on the dermis-epidermis junction, on the production of protein HSPs, on the firmness, the elasticity, the tone of the skin, regrowth of hair (eyelashes, eyebrows for example), etc.

Particularly and advantageously, in the context of the use according to the first aspect of the invention and of the ingredient according to the second aspect of the invention, the cyclic peptide(s) may be associated with at least one active agent adapted to act in a complementary manner on the properties of the epidermis and/or the *stratum corneum* (for example protective of the cutaneous barrier and/or moisturizing).

Such additional active agent may be chosen from the group comprising: phospholipids, the various ceramides, sphingosine, phytosphingosine, glycosphingolipids, cholesterol and its derivatives, sterols (in particular those of canola and soybean), fatty acids (including linoleic acid, palmitic acid, lipoic acid, thioctic acid), squalane (especially of olives), triglycerides (especially of coconut oil), lanolin, alcohols from lanolin, lanosterol, vitamin D3, tocopheryl nicotinate, various oils (especially, argan, rose, baobab), ascorbic acid, N-acetyl cysteine and N-acetyl-L-serine, vitamin B3compounds (such as niacinamide and nicotinic acid), panthenol, pseudofilaggrin, arginine, serine, PCA salts (pyrrolidone carboxylic acid), *Centella asiatica* leaf extract (titrated in madecassoside and asiaticoside), some plant extracts (roots of wild yam, chestnut, bud of cedar, solanaceae), extracts of plankton and yeast. The following actives sold by Sederma can also be mentioned: Venuceane^{™} (extract of the fermentation medium of *Thermus thermophilus*), Moist 24^{™} (hydroglycolic extract of *Imperata cylindrica* roots), Dermaxyl^{™} (association of ceramide 2 and of the peptide Pal-VGVAPG), Senestem^{™} (from *in-vitro* cell cultures of *Plantago lanceolata*), Ceramide 2^{™} (ceramide), Ceramide HO3^{™} (hydroxyceramide), Optim Hyal^{™} (oligosaccharides of acetylated glucuronic acids), Meiritage^{™} (association of extracts of *Bupleurum falcatum, Astragalus membranaceus* and *Atractylodes macrocephalea* roots), Revidrate^{™} (myristyl phosphomalate), Pacifeel^{™} (an extract of *Mirabilis jalapa*), Hydronesis^{™} (from the fermentation of *Salinococcus hispanicus*), NG unsaponifiables of shea butter^{™} and Citystem^{™} (from *in-vitro* cell culture of *Marrubium vulgare).*

Similarly, in a particular and advantageous manner, in the context of the use according to the first aspect of the invention and of the ingredient according to the second aspect of the invention, the cyclic peptide(s) can be associated with at least one active agent adapted to act on the reduction of sebum production by the sebocytes, the cyclic peptide(s) acting on the dermis, therefore on the reinforcement of the pore-supporting tissue and the additional active agent acting in reinforcement of the activity on sebum production. Thus, advantageously an ingredient is provided that is particularly active to beautify the skin's grain and therefore its radiance. Such additional active agent may be chosen from the group comprising: one or more pure molecules: an alkyl-phthalide or a plant extract comprising it, sulfur and its organic derivatives (for example S-carboxymethylcysteine), but also sulfur amino acids, cystine, cysteine, methionine, zinc and/or copper salts, for example the salts of L-pyrrolidone carboxylic acid (Cuivridone^{®} and Zincidone^{®} from Solabia), retinoic acid, vitamin B6, benzoyl peroxide, salicylic acid, ammonium lactate, aluminum hydroxy chloride, 10-hydroxydecanoic acid, glycine grafted on an undecylenic chain, such as that sold under the name Lipacide UG OR by the company Seppic, trialkyl citrate (C12-C13) sold under the name COSMACOL^{®}ECI by the company Sasol. Among the extracts of natural origin, there are in particular an argan oil, a clove extract, a *Serenoa serrulata* extract, a Ulmaire extract, a *Cinchona succirubra* bark extract, a Phellodendron extract, a meadowsweet extract, a *Sophora* extract, a *Laminaria* seaweed extract, a *Porphyridium cruentum* micro-algae extract enriched in zinc sold by Vincience under the name Algualane Zinc^{®}, and kaolin (white clay).

Actives of this type are also sold by Sederma, in particular: Biodermine^{™} (bacterial culture filtrate rich in peptides), Sebosoft^{™} (sebacic acid in a polyacrylate gel), Sebomine SB12^{™} (a combination of lactoferrin, glucose oxidase, lactoperoxidase and potassium thiocyanate), Normaseb^{™} (fermented casein filtrate), Yeast Biomembranes^{™}, Sebuless^{™} (an extract of *Syringa vulgaris* cell culture), Evermat^{™} (a combination of an *Enantia chlorantha* extract and oleanolic acid)) and ac.net ^{™} (a combination of oleanolic acid and nordihydroguaiaretic acid).

Likewise, in a particular and advantageous manner, in the context of the use according to the first aspect of the invention and of the ingredient according to the second aspect of the invention, the cyclic peptide(s) can be associated with at least one astringent active to "mechanically" tighten the pores to further improve the immediate smoothing and homogenizing effect of the cyclic peptides. Such additional active ingredient may be chosen from the group comprising as pure molecules: certain aluminum salts such as aluminum hydroxychloride, dihydroxy aluminum, alum, aluminum acetate, allantoin dihydroxy aluminum; zinc phenolsulfonate of Interchemical, zinc sulfate, zinc oxide, tannic acid, oxalic acid, citric acid, tartaric acid; as plant extracts: tannins, especially condensed or ellagic, extracts of horse chestnut, mauve, Hammamelis, oats, sweet almonds, *althaea officinalis* roots, burdock root, poison ivy, birch bark, horsetail, chamomile, scutellaria, cohosh roots, ulmaria, St. John's wort, willow, myrtle, a mixture of extracts of white ginger, horsetail, poison ivy, rosemary, yucca, extracts of acacia, elm, white willow, cinnamon, birch, meadowsweet, gentian, cucumber, walnut, ratanhia, grapefruit, extract of many fruits of the Rosaseae family such as prunella, loquat, but also apple, pear, quince, the mountain ash, the hawthorn; immature persimmon, green banana, species extracts of the genus Myrica, chicory roots, blackcurrant berries, currant, blueberry, cranberry, sea buckthorn and goji.

Preferably, said additional active agent is at least one alkyl phthalide chosen from the group comprising sedanenolide, sedanolide and 3-n-butylphthalide, preferably sedanenolide, or an extract of *Apium graveolens* seeds comprising a mixture of sedanenolide, sedanolide and 3-n-butylphthalide, preferably a supercritical CO₂ extract, and preferably an extract comprising a mixture of alkyl-phthalides comprising the sedanenolide as the major compound (in% by weight relative to the total weight of alkyl-phthalides). An ingredient of this type is described in the patent application WO2016157073. This active ingredient is advantageously capable of acting on two aspects, epidermis via a prodifferentiation of keratinocytes and on the production of sebum by the sebocytes. *In-vitro* and *in-vivo* results are given below in the description for this particularly advantageous combination.

The cyclic peptide(s) according to the invention may also be combined with at least one of the compounds chosen from vitamin B3 compounds, compounds such as niacinamide or tocopherol, retinoid compounds such as retinol, hexamidine, α-lipoic acid, resveratrol or DHEA, hyaluronic acid, peptides, especially N-acetyl-Tyr-Arg-O-hexadecyl, Pal-VGVAPG (SEQ ID NO: 10), Pal-KTTKS (SEQ ID NO: 1), Pal-GHK, Pal-KMO2K, Pal-GQPR (SEQ ID NO: 2) and Pal-K(P)HG, which are conventional active ingredients used in cosmetic or dermatological compositions.

According to a third aspect, the present invention provides a composition comprising the ingredient according to the second aspect of the invention described above, such a composition that can be used in cosmetics or for a topical medical application for the care of skin, its appendages and mucous membranes.

According to a fourth aspect, the present invention also provides a non-therapeutic method for improving the aesthetic appearance of the skin comprising topically applying to the skin an effective amount of a cosmetic composition comprising the active cosmetic ingredient according to the second aspect of the invention described above.

The cyclic peptides according to the invention may be optically pure or may consist of L or D isomers or a mixture thereof. The naturally occurring L-isomers may be preferred. The peptides may be found in the form of salts.

The present invention encompasses also cyclic peptide derivatives (with modification and/or addition of a chemical function but without change in the carbon skeleton) and analogues (with modification and/or addition of a chemical function but with additionally a change in the carbon skeleton), complexes with other species such as a metal ion (eg copper, zinc, manganese, magnesium, and others).

"Physiologically acceptable medium" means according to the present invention, without limitation, an aqueous or aqueous-alcoholic solution, a water-in-oil emulsion, an oil-in-water emulsion, a microemulsion, an aqueous gel, an anhydrous gel, a serum, a dispersion of vesicles or a powder.

"Physiologically acceptable" means that the compositions are suitable for topical use in contact with the skin and scalp of mammals and more particularly of human, without risk of toxicity, incompatibility, instability, allergic response, among others. This "physiologically acceptable medium" forms what is usually called the excipient of the composition.

The cyclic peptides used according to the invention may be solubilized in a lipophilic or hydrophilic matrix with, if appropriate, a solubilizer, depending on the future end application.

A composition according to the invention can be applied to all parts of the body, and more specifically according to the indication recommended on the face, body, neckline or scalp, in any form or vehicle known to the skilled persons in the art, particularly in the form of a solution, dispersion, emulsion, paste or powder, individually or in premix or be conveyed individually or premixed with vectors such as macrocapsules, microcapsules or nanocapsules, macrospheres, microspheres, or nanospheres, liposomes, oleosomes or chylomicrons, macroparticles, microparticles or nanoparticles, macro-sponges, micro-sponges or nanosponges, microemulsions or nanoemulsions, or adsorbed on polymers powdery organic, talcs, bentonites, spores or exines and other mineral or organic carriers.

In cosmetics in particular, applications can be proposed especially in the skincare ranges of the face, body, hair, and scalp and in make-up care ranges.

In general, the cyclic peptides according to the present invention can be used in any form, in a bound form, incorporated or adsorbed on macro-, micro-, and nanoparticles, or on macro-, micro- and nanocapsules, for the treatment of textiles, natural or synthetic fibers, wools, and any material intended to come into contact with the skin and which may be used in clothing, underwear, day or night, handkerchiefs, or the tissues, in order to exert its cosmetic or therapeutic effect through this skin/textile contact and to allow a continuous topical delivery.

The CTFA ("International Cosmetic Ingredient Dictionary & Handbook" (18th Edition, 2018) published by "The Cosmetic, Toiletry, and Fragrance Association, Inc.", Washington, DC) describes a wide variety, without limitation, of cosmetic ingredients usually used in the skincare and scalp care industry, which are suitable for use as additional ingredients in the compositions of the present invention.

Other additional skin care actives that are particularly useful can be found in Sederma's commercial literature and at www.sederma.com or www.crodarom.fr.

The following commercial actives can also be mentioned as examples: betain, glycerol, Actimoist Bio 2^{™} (Active organics), AquaCacteen^{™} (Mibelle AG Cosmetics), Aquaphyline^{™} (Silab), AquaregulK^{™} (Solabia), Carciline^{™} (Greentech), Codiavelane^{™} (Biotech Marine), Dermaflux^{™} (Arch Chemicals, Inc), Hydra'Flow^{™} (Sochibo), Hydromoist L^{™} (Symrise), RenovHyal^{™} (Soliance), Seamoss^{™} (Biotech Marine), Argireline^{™} (nom commercial de l'acétyl hexapeptide-3 from Lipotec), spilanthol or an extract of *Acmella oleracea* known under the trade name Gatuline Expression^{™}, an extract of *Boswellia serrata* known under the name Boswellin^{™}, Deepaline PVB^{™} (Seppic), Syn-AKE^{™} (Pentapharm), Ameliox^{™}, Bioxilift^{™} (Silab), PhytoCellTec^{™}Argan (Mibelle), Papilactyl D^{™} (Silab), Preventhelia^{™} (Lipotec), or one or more of the following active ingredient sold by Sederma : Subliskin^{™}, Venuceane^{™}, Moist 24^{™}, Vegesome Moist 24^{™}, Essenskin^{™}, Juvinity^{™}, Revidrat^{™}, Resistem^{™}, Chronodyn^{™}, Kombuchka^{™}, Chromocare^{™}, Calmosensine^{™}, Glycokin factor S^{™}, Biobustyl^{™}, Idealift^{™}, Ceramide 2^{™}, Ceramide A2^{™}, Ceramide HO3^{™}, Legance^{™}, Intenslim^{™}, Prodizia^{™}, Beautifeye^{™}, PacifeelTM, Zingerslim^{™}, Meiritage^{™}, Senestem^{™}, Sebuless^{™}, Majestem^{™}, Apiscalp^{™}, Rubistem^{™}, CitystemTM, Neonyca^{™}, NG Insaponifiables de Beurre de Karité^{™}, Majestem^{™}, Hydronesis^{™}, Poretect^{™} and Crystalide^{™}, or mixture thereof.

Among plant extracts (in the form of conventional extracts or prepared by an *in vitro* method) that can be combined with the cyclic peptide(s) according to the invention, there may more particularly be mentioned extracts of Ivy, in particular English Ivy (*Hedera helix*), of *Bupleurum chinensis,* of *Bupleurum falcatum,* of arnica (*Arnica montana L*), of rosemary (*Rosmarinus officinalis N*), of marigold (*Calendula officinalis*), of sage (*Salvia officinalis L*), of ginseng (*Panax ginseng*), of ginko biloba, of St.-John's-Wort (*Hyperycum perforatum*), of butcher's-broom (*Ruscus aculeatus L*), of European meadowsweet (*Filipendula ulmaria L*), of big- flowered Jarva tea (*Orthosiphon stamincus benth*), of artichoke (*Cynara scolymus*), of algae (*Fucus vesiculosus*), of birch (*Betula alba*), of green tea, of cola nuts (*Cola nipida*), of horse-chestnut, of bamboo, of *Centella asiatica,* of heather, of fucus, of willow, of mouse-ear, of escine, of cangzhu, of *chrysanthellum indicum,* of the plants of the *Armeniacea* genus, *Atractylodis platicodon, Sinnomenum, Pharbitidis, Flemingia,* of *Coleus* such as C. *Forskohlii, C. blumei, C. esquirolii, C. scutellaroides, C. xanthantus* and C. *Barbatus,* such as the extract of root of *Coleus barbatus,* extracts of *Ballote,* of *Guioa,* of *Davallia,* of *Terminalia,* of *Barringtonia,* of *Trema,* of *antirobia, cecropia, argania, dioscoreae* such as *Dioscorea opposita* or Mexican, extracts of *Ammi visnaga,* of *Siegesbeckia,* in particular *Siegesbeckia orientalis,* vegetable extracts of the family of *Ericaceae,* in particular bilberry extracts (*Vaccinium angustifollium*) or *Arctostaphylos uva ursi, aloe vera,* plant containing sterols (e.g., phytosterol), Manjistha (extracted from plants of the genus *Rubia,* particularly *Rubia cordifolia*), and Guggal (extracted from plants of the genus *Commiphora,* particularly *Commiphora mukul*), kola extract, chamomile, red clover extract, Piper methysticum extract (Kava Kava^{™} from Sederma), *Bacopa monieri* extract (Bacocalmine^{™} from Sederma) and sea whip extract, extracts of *Glycyrrhiza glabra,* of mulberry, of *melaleuca* (tea tree), of *Larrea divaricata,* of *Rabdosia rubescens,* of *Euglena gracilis,* of *Fibraurea recisa Hirudinea,* of *Chaparral Sorghum,* of sun flower extract, of *Enantia chlorantha,* of Mitracarpe of *Spermacocea* genus, of *Buchu barosma,* of *Lawsonia inermis L.,* of *Adiantium capillus-veneris L., of Chelidonium majus,* of *Luffa cylindrica,* of Japanese Mandarin (*Citrus reticulata Blanco* var. *unshiu*), of *Camelia sinensis,* of *Imperata cylindrica,* of *Glaucium Flavum,* of *Cupressus sempervirens,* of *Polygonatum multiflorum,* of *loveyly hemsleya,* of *Sambucus nigra,* of *Phaseolus lunatus,* of *Centaurium,* of *Macrocystis pyrifera,* of *Turnera diffusa,* of *Anemarrhena asphodeloides,* of *Portulaca pilosa,* of *Humulus lupulus,* of *Coffea arabica,* of *Ilex paraguariensis,* or of *Globularia cordifolia,* of *Albizzia julibrissin,* of *Oxydendron arboretum,* of *Zingimber zerumbet smith,* of *Astragalus membranaceus,* of *Atractylodes macrocephalae,* of *Plantago lanceolata,* of *Leontopodium alpinum,* of *Mirabilis jalapa,* of *Marrubium vulgare,* or of orchids.

The compositions of the present invention may include one or more additional peptides, including, without limitation, di-, tri-, tetra-, penta-and hexapeptides and their derivatives. According to a particular embodiment, the concentration of the additional peptide, in the composition, ranges from 1x10⁻⁷% and 20%, preferably from 1x10⁻⁶% and 10%, preferably between 1x10⁻⁵% and 5% by weight. The term "peptide" refers here to peptides containing 10 amino acids or less, their derivatives, isomers and complexes with other species such as a metal ion (e.g. copper, zinc, manganese, magnesium, and others). The term "peptides" refers to both natural peptides and synthetic peptides. It also refers to compositions that contain peptides and which are found in nature, and/or are commercially available.

Suitable dipeptides for use herein include but are not limited to Carnosine (βAH), YR, VW, NF, DF, KT, KC, CK, KP, KK, TT, PA, PM or PP.

Suitable tripeptides for use herein include, but are not limited to RKR, HGG, GKH, GHK, GGH, GHG, KFK, KAvaK, KβAK, KAbuK, KAcaK, KPK, KMOK, KMO₂K (MO₂ being a di-oxygenated sulfoxide methionine), KVK, PPL, PPR, SPR, QPA, LPA or SPA.

Suitable tetrapeptides for use as additional peptides herein include but are not limited to RSRK (SEQ ID NO: 11), GQPR (SEQ ID NO: 12), KTFK (SEQ ID NO: 13), KTAK (SEQ ID NO: 14), KAYK (SEQ ID NO: 15) or KFYK (SEQ ID NO: 16). Suitable pentapeptides include but are not limited to KTTKS (SEQ ID NO: 17). Suitable hexapeptides include but are not limited to GKTTKS (SEQ ID NO: 18) and VGVAPG (SEQ ID NO: 19).

Other suitable peptides for use as additional peptides herein include but are not limited to: lipophilic derivatives of peptides, preferably palmitoyl (Pal) derivatives or myristoyl (Myr), and metal complexes as aforementioned (e.g. copper complex of the tripeptide HGG). Preferred dipeptides include for example N-Palmitoyl-β-Ala-His, N-Acetyl-Tyr-Arg-hexadecylester (Calmosensine^{™}, Idealift^{™} from Sederma), Pal-RT or Pal-KT (Sederma). Preferred tripeptide derivatives include for example Pal-GKH and Pal-GHK (from Sederma), the copper derivative of HGG (Lamin^{™} from Sigma), Pal-GHK, Lipospondin (N-Elaidoyl-KFK) and its analogs of conservative substitution, N-Acetyl-RKR-NH₂ (Peptide CK+), N-Biot-GHK (from Sederma), Pal-KAvaK, Pal-KβAlaK, Pal-KAbuK, Pal-KAcaK, or Pal-KMO₂K (Matrixyl^{®}synthe'6^{®} from Sederma), Pal-KVK (Syn-Coll^{™} of DSM), and derivatives thereof.

Mention may also be made here of the anti-aging tripeptides of general formula X-Pro*-Pro*-Xaa-Y described in WO2015181688application with Xaa selected from Leu, Arg, Lys, Ala, Ser, and Asp, at the N-terminus , X chosen from H, -CO-R1 and -SO2 -R1 and at the C-terminal end Y chosen from OH, OR1, NH 2, NHR1 or NR1R2, R1 and R2 being, independently of one another, chosen from a alkyl, aryl, aralkyl, alkylaryl, alkoxy and aryloxy group, which may be linear, branched, cyclic, polycyclic, unsaturated, hydroxylated, carbonylated, phosphorylated and/or sulfurized, said group possibly possessing in its backbone a heteroatom particularly O, S and/or or N, and Pro* corresponding to Proline, an analogue or derivative thereof; comprising, for example, Myr-PPL-OH and Myr-PPR-OH.

Here can further be cited also the propigmenting and/or pro-mec dipeptides and tripeptides of general formula X-(Xaa₁)n-Pro*-Xaa₂-Y disclosed in WO2014/080376, with n=0, 1 or 2, Xaa₁ an hydrophobic aminoacid selected from Ala, Val, Met, Leu, Iso, Phe, Pro, and analogs and derivatives thereof; or a polar aminoacid selected from Ser, Thr, Tyr, Asp, Glu and analogs and derivatives thereof; and when n=2 the two aminoacids Xaa₁ being the same or different; Xaa₂ being an hydrophobic aminoacid selected from Ala, Val, Met, Leu, Iso, Phe, and analogs and derivatives thereof, or a basic aminoacid selected from Arg, Lys, His, and analogs and derivatives thereof; at the N terminal end X being selected from H, -CO-R₁ and -SO₂-R₁; at the C terminal end Y being selected from OH, OR₁, NH₂, NHR₁ or NR₁R₂; R₁ and R₂ being, independently from each other, selected from an alkyl, aryl, aralkyl, alkylaryl, alkoxy et aryloxy group, that can be linear, branched, cyclic polycyclic, saturated, unsaturated, hydroxylated, carbonylated, phosphorylated and/or sulfured, said group having or not an O, S and/or N heteroatom in its skeleton and Pro* corresponding to a Proline, analog or derivative thereof; comprising for example the following peptides Pal-SPR-OH, Pal-PPR-OH, Pal-QPA-OH, Pal-LPAOH, Myr-SPA-OH, Pal-PM-OH, Pal-PA-OH and Pal-PP-OH.

Suitable tetrapeptide derivatives for use as additional peptides according to the present invention include, but are not limited to, Pal-GQPR (SEQ ID NO: 2) (from Sederma) and Pal-KTFK (SEQ ID NO: 20) or Ela-KTFK (SEQ ID NO: 21), Ela-KTAK (SEQ ID NO: 22), Ela-KAYK (SEQ ID NO: 23) or Ela-KFYK (SEQ ID NO: 24). Suitable pentapeptide derivatives for use as additional peptides herein include, but are not limited to, Pal-KTTKS (SEQ ID NO: 1) (available as Matrixyl^{®} from Sederma), Pal-YGGFXaa (SEQ ID NO: 25) with Xaa being Leu or Pro, or mixtures thereof. Suitable hexapeptide derivatives for use herein include, but are not limited to, Pal-VGVAPG (SEQ ID NO: 10), Pal-GKTTKS (SEQ ID NO: 26), Pal-HLDIIXaa with Xaa being Trp, Phe, Tyr, Tic, 7-hydroxy-Tic ou Tpi (SEQ ID NO: 27) and derivatives thereof. The mixture of Pal-GHK and Pal-GQPR (SEQ ID NO: 2) (Matrixyl^{®} 3000, Sederma) can also be mentioned.

The preferred compositions commercially available containing a tripeptide or a derivative include Biopeptide-CL^{™}, Maxilip^{™}, Biobustyl^{™}, Procapil^{™} and Matrixyl^{®}synthe'6^{®} of Sederma. The compositions commercially available preferred sources of tetrapeptides include Rigin^{™}, Eyeliss^{™}, Matrixyl^{®} Reloaded and Matrixyl 3000^{®} which contain between 50 and 500 ppm of Pal-GQPR (SEQ ID NO: 2) and an excipient, proposed by Sederma.

The following sold peptides can be mentioned as well as additional active ingredients:
- Vialox^{™} (INCI name = Pentapeptide-3 (synthetic peptide comprising alanine, arginine, isoleucine, glycine and proline)), Syn-ake^{™} (β-Ala-Pro-Dab-NH-Bzl) or Syn-Coll^{™} (Pal-Lys-Val-Lys-OH) sold by Pentapharm;
- Argireline^{™} (Ac-Glu-Glu-Met-Gln-Arg-Arg-NH₂ (INCI name = Acetyl hexapeptide-3) (SEQ ID NO: 28), Leuphasyl^{™} (Tyr-D-Ala-Gly-Phe-Leu) (SEQ ID NO: 29), Aldenine^{™} (Gly-His-Lys), Trylagen^{™} (INCI name = Pseudoalteromonas Ferment Extract, Hydro lyzed Wheat Protein, Hydro lyzed Soy Protein, Tripeptide-10 Citrulline (reaction product of Citrulline and Tripeptide-10 (synthetic peptide constituted of aspartic acid, isoleucine and lysine)), Tripeptide-1), Eyeseryl^{™} (Ac-β-Ala-His-Ser-His)(SEQ ID NO: 30), Serilesine^{™} (Ser-Ile-Lys-Val-Ala-Val) (SEQ ID NO 31) or Decorinyl^{™} (INCI name: Tripeptide-10 Citrulline = reaction product of Citrulline and Tripeptide-10 (synthetic peptide constituted of aspartic acid, isoleucine and lysine) sold by Lipotec;
- Collaxyl^{™} (Gly-Pro-Gln-Gly-Pro-Gln (SEQ ID NO 32)) or Quintescine^{™} (Cys-Gly) sold by Vincience;
- Cytokinol^{™}LS (casein hydrolysate) sold by Les Laboratoires Serobiologiques/Cognis;
- Kollaren^{™} (Gly-His-Lys), 1P2000^{™} (Pal-Val-Tyr-Val) or Meliprene^{™} (INCI name = Monofluoroheptapeptide-1: reaction product of acetic acide and a synthetic peptide comprising arginine, glycine, glutamic acid, histidine, norleucine, p-fluorophenylalanine and tryptophan) sold by l'lnstitut Européen de Biologie Cellulaire;
- Neutrazen^{™} (Pal-His-D-Phe-Arg-NH₂) sold by Innovations; or
- BONT-L-Peptide^{™} (INCI name = Palmitoyl Hexapeptide-19: reaction product of palmitic acid and Hexapeptide-19 (synthetic peptide constituted of asparagine, aspartic acid, lysine and methionine), Timp-Peptide^{™} (INCI name = Acetyl Hexapeptide-20: reaction product obtained by acetylation of Hexapeptide-20 (synthetic peptide constituted of alanine, glycine, lysine, valine and proline) or ECM Moduline^{™} (INCI name = Palmitoyl Tripeptide-28: reaction product of palmitic acid and Tripeptide-28 (synthetic peptide constituted of arginine, lysine and phenylalanine) sold by lnfinitec Activos.

According to the invention, by "topical treatment" or "topical use" is meant an application that is intended to act at the place where it is applied: skin, muquous membrane, skin appendages.

The cyclic peptide(s) or the composition according to the invention can be applied locally to the targeted areas, for example using a canula-type applicator.

The "effective" amount depends on a variety of factors, such as age, condition of the patient, severity of the disorder or condition and the way of administration. An effective amount means a non-toxic amount sufficient to achieve the desired effect.

In a cosmetic composition according to the invention, the cyclic peptide(s) to be present in an effective amount, are generally in proportions of between 0.000001% and 5% relative to the total weight of the composition, preferably between 0.00001% and 0.1%, more preferably between about 0.0005 and 0.005%, depending on the destination of the composition and the desired effect more or less pronounced.

All percentages and ratios used in this application are by weight of the total composition and all measurements are made at 25°C unless otherwise specified.

As for example, for a cosmetic facial treatment, the European Cosmetics Directive has set a standard application amount of a cream of 2.72 mg/cm²/day/person and for a lotion for the body of 0.5mg/cm²/day/person.

According to other features, the cosmetic treatment method according to the invention may be associated with one or more other treatment methods for the skin, such as, for example, light therapy, heat or aromatherapy treatments.

According to the invention, it is possible to propose devices with several compartments or kits intended for the implementation of the method described above, and which could include, by way of example, and without being limiting, in a first compartment a composition containing at least the active according to the invention and in a second compartment an additional excipient and/or active, the compositions contained in said first and second compartments being here considered as a combination composition for simultaneous, separate or spread over time use, especially in one of the treatments defined above.

A composition according to the third aspect of the invention is suitable for a therapeutic treatment, in particular a treatment of the skin, in particular a skin deficient in molecules constituting the dermal extracellular matrix.

### DETAILED DESCRIPTION

The following examples describe and illustrate certain aspects of the invention. They should not be perceived as limiting the disclosure, as they provide mainly information useful for its understanding and implementation. The detailed description is made with reference to the accompanying drawings in which:
FIG. 1 shows by way of illustration a cyclic peptide according to the invention, the *Cyclo*(-Met-Leu-Ile-Pro-Pro-Phe-Phe-Val-Ile) (SEQ ID NO: 5);
FIG. 2 is a table showing the cyclic peptide composition of the mixture of Example A); and
FIG 3 shows a graph of deformation of the skin as a function of time obtained using a cutometer used for *in-vivo* evaluation.

### A) Example of manufacturing of cyclic peptides according to the invention

Flax seeds (*Linum usitatissimum* L.) are milled in an oil press and then extracted with aqueous ethanol. The mixture is then stirred about 1 hour under blades or any other suitable means. The suspension is allowed to separate into two phases for 3 to 5 days. The fractions of spent oil (emptied of cyclic peptides) and ethanol are separated. The oily fraction is removed. The ethanol is evaporated under vacuum. The solid residue constituting the mixture of cyclic peptides according to the invention is collected, dried and grounded into a fine powder.

The mixture obtained by this method essentially consists of cyclic peptides and for the rest of lipids (fatty acids and triglycerides).

The cyclic peptides were separated and quantified by HPLC in the system: C18Column, with a 5 mM ammonium acetonitrile/formate gradient (detection at 214 nm that can be supplemented by mass spectrometry analysis).

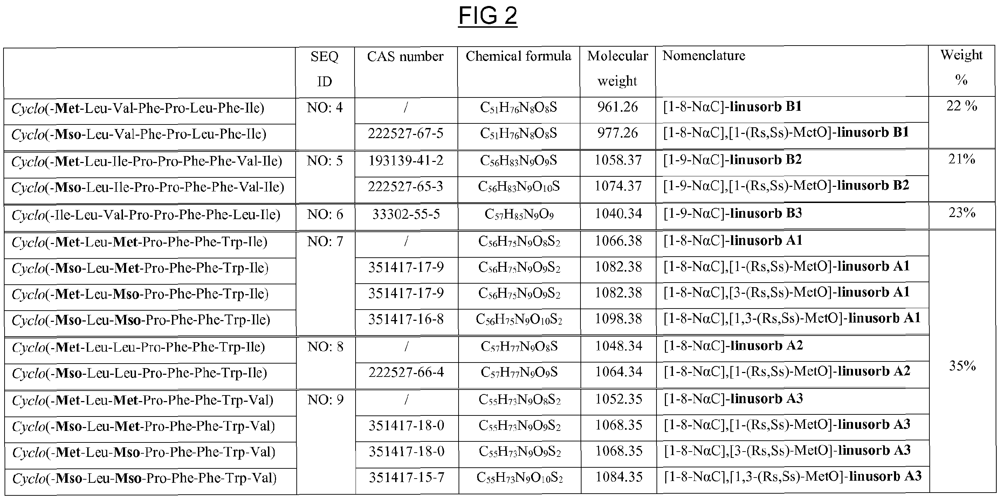

FIG. 2 gives the qualitative and quantitative composition of the mixture:
- three cyclic peptides predominate: Linusorb B1 (SEQ ID NO: 4), Linusorb B2 (shown for illustrative purposes in FIG. 1 and SEQ ID NO: 5) and Linusorb B3 (SEQ ID NO: 6); they represent, respectively, 22%, 21% and 23%, thus a total of 65% by weight relative to the total weight of the mixture of cyclic peptides;
- three other cyclic peptides are in a smaller proportion, Linusorb A1 (SEQ ID NO: 7), Linusorb A2 (SEQ ID NO: 8) and Linusorb A3 (SEQ ID NO: 9), representing 35% by weight relative to the total weight of the mixture of cyclic peptides.

The Linusorb B1, B2, A1, A2 and A3 have in their sequence a methionine which can be oxidized according in particular to the origin of the seeds. In the mixture described above, part of the methionines is oxidized to sulphoxides, representing approximately 40% by weight relative to the total weight of cyclic peptides.

Other examples of cyclic peptides according to the invention and of their preparation have also been described in patent applications WO200979792 and WO2013091070.

### B) Example of preparing cosmetic or topical therapeutic active ingredients according to the invention

### 1/ Preparing an ingredient based on a mixture of cyclic peptides

Active: 1000 ppm of the mixture of cyclic peptides according to the invention, whose preparation is described in A) above.

Excipient: a fatty excipient, for example an esterified oil of Caprylic/Capric Triglyceride type. Procedure: hot solubilization of the active and under middle stirring in the fatty excipient.

### 2/ Preparing an ingredient based on an association of a cyclic peptide mixture according to the invention and a supercritical CO₂ extract of Apium graveolens

As disclosed above, the mixture according to the invention of cyclic peptides can advantageously be combined with a supercritical CO₂ extract of celery seeds (*Apium graveolens*).

An extract of this type, described by the Applicant in the patent application WO 2016/157073, is prepared in the following manner: the celery seeds are crushed to obtain a particle size powder around 800 µm. This powder is then extracted with supercritical CO₂ at 90 bars of pressure and at 40°C. Residual water that may be present in the final extract is then removed. The extract is in the form of an oily liquid comprising 71% by weight of total phthalides comprising 3 alkyl phthalides: 10% of 3-n-butylphthalide, 30% of sedanolide and 60% of sedanenolide, in % by weight relative to the total weight of phthalides.

Actives: 500 ppm of the mixture of cyclic peptides according to the invention, the manufacture of which has been described in A) above + 700 ppm of the supercritical CO₂ extract of celery seeds (comprising at the end 500 ppm of total phthalides).

Excipient: a fatty excipient (for example an esterified oil of Caprylic/Capric Triglyceride type) in the presence of a surfactant (for example a sorbitan trioleate).

Procedure: the mixture of cyclic peptides according to the invention manufactured in A) is hot solubilized and under medium stirring in a portion of the fatty excipient, in the presence of the surfactant. Without cooling, the remainder of the fatty excipient is added while continuing stirring to room temperature. The supercritical CO₂ extract of celery seeds is then solubilized in the mixture with medium agitation.

As an example, and for the description of the *in-vivo* tests and the galenic of the point D), it is these ingredients which have been used (between 0.5% and 10% of the ingredient 1 or the ingredient 2 in a cosmetic composition applicable to the skin).

### C) In-vitro EFFICACY TESTS

They were conducted in three distinct phases developed below:
1) Effect of the mixture of cyclic peptides according to the invention (as prepared in A) above)
2) Comparative tests: pure cyclic peptides against the mixture of cyclic peptides
3) Effect of the combination of the mixture of cyclic peptides and the CO₂ supercritical extract of celery seeds

### 1/ Interest of cyclic peptides according to the invention

### 1.1/ Densifying effect of the dermis / reinforcement of supporting tissues

The dermis of an 80-year-old person contains four times more fragmented collagen than people aged 20-30 who have longer fibers. This fragmentation leads to reducing up to 80% of the interactions that the cells maintain with their matrix. With age, dermal fibroblasts produce less supportive proteins, including less collagen-I, the most abundant protein in the skin, and also elastin.

This explains the structural and functional decline of the skin that becomes less dense, less organized, less dynamic. The weakening of the qualities of the supporting tissue causes a decrease in the visco-elastic characteristics of the skin: firmness, elasticity and tone are thus reduced by about 13% per decade.

A cosmetic active capable of stimulating the production of components of the dermis matrix is therefore particularly searched.

### Principe:

Two complementary methods were used. Human fibroblasts of dermal origin (HDF) were seeded in a culture medium providing them the growth factors necessary for their physiology and their multiplication. When the cells reached confluency, they received the products to be tested. After three days of contact, the culture media were removed, and the amount of elastin produced by the cells was assayed by ELISA (n = 5) and compared with that obtained with the product solvent (control) (Table 1 below).

In a second series of experiments, after a contact of seven days, the elastin fibers produced by the cells were highlighted by fluorescence immunocytology (IMF, n = 3) on the fixed mats and quantified by analysis of the images. The results are expressed in arbitrary fluorescence units/10⁷ cells.

The number of cells was assessed using the Hoescht 33258 method, which marks the DNA. A variance study and a Student t-test for non-paired series were performed to evaluate the significance of the results.

The production of collagen-I was demonstrated with the same methods (Table 2).

### Results:

**Table 1: Variation of elastin production by HDF after contact with the mixture of cyclic peptides according to the invention.**

| **Concentrations** | **Elastin (ELISA)** | **Elastin (IMF)** |
|---|---|---|
| | **Variation % / control** | **Variation % / control** |
| **Control** | Reference | Reference |
| **10 ppm** | + 40%; *p<0.01* | + 295%; *p<0.01* |
| **15 ppm** | + 142%; *p<0.01* | + 519%; *p<0.01* |

**Table 2: Variation of collagen-I production by HDF after contact with the mixture of cyclic peptides according to the invention.**

| **Concentrations** | **Collagen I % (ELISA)** | **Collagen I % (IMF)** |
|---|---|---|
| | **Variation % / control** | **Variation % / control** |
| **Control** | Reference | Reference |
| **10 ppm** | + 60%; *p<0.01* | + 219%; *p<0.01* |
| **15 ppm** | + 115%; *p<0.01* | + 349%; *p<0.01* |

These data indicate the potential of the mixture of cyclic peptides according to the invention in optical reinforcement of the tissues supporting the dermis, for more firmness and elasticity.

### 1.2/ Strengthening of the dermo-epidermal junction (DEJ)

Laminins are important at the level of the DEJ. They are part of the basal layer and ensure the proper anchoring of basal keratinocytes on the basal membrane and are responsible for the flexibility of the epidermis. In addition, they stimulate the proliferation of keratinocytes, allowing them to engage in differentiation. On older cells they are no longer replaced as efficiently as on young cells, hence the interest of stimulating their biosynthesis for a better renewal.

The increase of collagen IV syntheses is also searched. It helps restore/strengthen the DEJ. Collagen IV forms a two-dimensional network and is one of the major components of the DEJ.

The reduction of protein synthesis with age is felt at the level of the DEJ. Thus, collagen IV is more fragmented and at the same time less produced, as well as are laminins, which in some areas leads to an alteration of the DEJ and a poorer communication between melanocytes, keratinocytes and DEJ and less flexibility of the system. The interest of stimulating the synthesis of these two proteins therefore clearly appears.

### Principe:

The mixture of cyclic peptides according to the invention in solution in the culture medium was brought into contact with HDF. An assay of laminins and collagen IV was made on the media; the result is reduced to the number of cells present on the mat.

### Results:

**Table 3: Variation of laminin production by HDF after contact with different concentrations of the mixture of cyclic peptides according to the invention; Elisa (n=4).**

| **Concentrations** | **Variation % / control** |
|---|---|
| **Control** | Reference |
| **5 ppm** | +27%*; p<0.01* |
| **10 ppm** | +43%*; p<0.01* |
| **12.5 ppm** | +62%*; p<0.01* |
| **15 ppm** | +76%*; p<0.01* |

The mixture of cyclic peptides according to the invention positively modulates the production of laminins. The effect is clear from 5 ppm and reaches +76% (*p*<*0.01*).

**Table 4: Variation of collagène IV production by HDF after contact with different concentrations of the mixture of cyclic peptides according to the invention; Elisa (n=4).**

| **Concentrations** | **Variation % / control** |
|---|---|
| **Control** | Reference |
| **5 ppm** | + 37.6; *p*<*0.01* |
| **10 ppm** | + 44.1; *p<0.01* |

The mixture of cyclic peptides according to the invention positively modulates the production of collagen IV. The effect is clear from 5ppm and reaches + 44.1% (*p<0.01*).

The mixture of cyclic peptides according to the invention thus stimulates the synthesis of laminins and IV collagen favoring better anchoring of basal keratinocytes to the DEJ, their proliferation/differentiation and a better anchoring of the DEJ on the elastic components of the dermis.

### 1.3/ Decrease of inflammation mediators

Sensitive and irritated skin is characterized by an abnormally high secretion of cytokines, pro-inflammatory peptides (IL-8, IL-6 for example) and pro-inflammatory lipids (PGE-2 for example). In addition, inflammation mediators, IL-6 and PGE-2, are known to induce premature aging phenomena *via* micro-inflammations.

It is thus searched in cosmetic formulations to integrate actives to reduce the production of IL-8, IL-6 and PGE2, so as to reduce inflammatory response and slow skin aging.

To test the active ingredients, skin cells were cultured under mild stress conditions (application of UVB radiation) to mimic micro-inflammation. In this situation a significant decrease in mediators of inflammation, IL-8, IL-6 and PGE-2, will be interpreted in the sense of an anti-inflammatory action.

### Principe:

HDF are grown until a confluent mat is obtained. At this stage, the HDF are put in contact with the test products for 24 hours, then the mats were irradiated with UVB and brought back into contact with the products to be tested for 24 hours. The amounts of PGE-2 and IL-8 synthesized were measured in culture supernatants by ELISA assay. The number of cells was evaluated in order to normalize the data. A study of the variances and a Student t-test for non-paired series were performed to evaluate the significance of the results.

### Results:

**Table 5: Variation in the amount of PGE₂ and IL-8 produced by fibroblasts after contact with different concentrations of the mixture of cyclic peptides according to the invention; (n=3)**

| **Concentrations** | **PGE2 (ELISA)** | **IL-8 (ELISA)** |
|---|---|---|
| | **Variation % / control** | **% de variation / control** |
| **Control** | Reference | Reference |
| **12.5 ppm** | -50; *p<0.01* | -30; *p<0.05* |
| **15 ppm** | -79; *p*<*0.01* | -45; *p<0.01* |

### Principe:

Human keratinocytes (HK) were cultured until a confluent mat is obtained. At this stage, they were put in contact with the test products for 24 hours, then the mats were irradiated with UVB and brought back into contact with the products to be tested for 24 hours. The amounts of PGE-2 and IL-8 synthesized were measured in culture supernatants by ELISA assay. The number of cells was evaluated in order to normalize the data. A study of the variances and a Student t-test for non-paired series were performed to evaluate the significance of the results.

### Results:

**Table 6: Variation of the amount of PGE₂ and IL-8 produced by keratinocytes after contact with different concentrations of the mixture of cyclic peptides according to the invention; (n=3)**

| **Concentrations** | **PGE2 (ELISA)** | **IL-8 (ELISA)** |
|---|---|---|
| | **Variation % / control** | **% de variation / control** |
| **Control** | Reference | Reference |
| **5 ppm** | -39; *p<0.05* | -31; *p<0.05* |
| **15 ppm** | -66; *p*<*0.01* | -35; *p<0.01* |

Thus, advantageously, the mixture of cyclic peptides according to the invention strongly and significantly reduces the two pro-inflammatory messengers in the two types of cells.

### 1.4/ Reduction of sebum production by sebocytes

The skin has many pores on its surface whose function is to remove excess sebum and impurities from the skin such as dead skin cells and sweat. Oily skin is associated with too much sebum production by the sebocytes. Too much sebum causes changes in the properties of the skin and scalp, for example by increasing the formation of pimples, blackheads and obstructing the pores which will then expand, become more visible and make the skin grain irregular.

An anti-seborrheic cosmetic active will oppose this evolution by reducing the production of sebum, which will have the effect of tightening the pores of the skin, to smooth and reduce the fat/shine appearance with an irregular grain, especially characteristic of oily skin.

**Principe:** sebocytes were seeded in their growth medium. At confluency, the cells are brought into contact with the cyclic peptide mixture according to the invention for 48 hours. After removal of the media, the cell mats are incubated with the intracellular lipid marker Red Nile, which allows the amount of lipids present in the cells to be estimated by measuring the fluorescence emitted. The viability estimation is performed in parallel on the same mats using a fluorescent dye.

### Results:

**Table 7: Modulation of the lipid synthesis in sebocytes after contact with different concentrations of the mixture of cyclic peptides according to the invention; (n=3)**

| **Concentrations** | **Variation % / control** |
|---|---|
| **Control** | Reference |
| **10 ppm** | *-33* %; *p<0.05* |
| **15 ppm** | -27 %; *p<0.05* |

These results show that exposure of the sebocytes to the mixture of cyclic peptides according to the invention makes it possible to reduce the amount of lipids in these sebum-producing cells.

The mixture of cyclic peptides according to the invention can therefore be used to treat skin disorders associated with oily skin or oily prone skin, such as the shiny, glossy appearance, the size and the number of pores, to give the skin a smoother appearance, uniform, more harmonious.

### 1.5/ Cross-talking keratinocytes/fibroblasts (tests in conditioned medium)

### a/ Preliminary test:

### Principe:

HK were cultured to confluency and received different doses of the mixture of cyclic peptides according to the invention or nothing (solvent control) for 24 hours. The cells, once rinsed, were then irradiated with ultraviolet B, so as to create a slight oxidative stress, and returned to culture for 24 hours. A non-irradiated HK culture served as a negative control. The culture medium of these stressed HK (conditioned medium) were removed and then deposited on human fibroblasts in order to evaluate the collagen-I production (by IMF) and the production of MMP1 (by ELISA) that can be induced by this conditioned medium.

### Results:

This test confirmed the increase in collagen-I and MMP1 production by fibroblasts in the presence of stratifin.

### b/ "cross-talking" test

### Principe:

HK were prepared as before, then received the cyclic peptide mixture according to the invention or its solvent as a negative control for 24 hours and were irradiated with type B ultraviolet in a neutral buffer to increase the production of the stratifin protein. The latter was then assayed by the Western Blot technique in culture media.

### Results:

With this test, it was demonstrated that the mixture of cyclic peptides according to the invention has the capacity to reduce the production of stratifin by HK subjected to UVB irradiation (-55% at 10ppm; *p>0.* 01) and consequently to strongly limit the effects induced by this protein on fibroblasts (decrease of collagen synthesis and increase of proteases of dermal proteins).

The mixture of cyclic peptides therefore has a protective effect with regard to the detrimental effects of stratifin on collagen I and MMP1.

### 1.6/ Action on mitochondrial proteins

Each mammalian cell comprises about 1500 mitochondria which provide the energy necessary for the functioning, the syntheses, the defense, and more generally the cutaneous homeostasis. Age-related mitochondrial DNA mutations can weaken the respiratory chain and increase well-known oxygen radical species known as a major factor in aging. There are also links between the decline of essential compounds for mitochondria and failures in mitochondrial energy production, dysfunctions or even pathologies. This phenomenon accelerates the aging of the organs and the skin in particular. Mitochondria require 1,200 proteins: 99% are encoded by the nuclear DNA, only 13 proteins, all important for respiration and ATP production, are designed in the mitochondria. Age or exogenous reasons (solar stress, alcohol, pollutants, drugs ...) reduce the supply of proteins from the cytoplasm, produce defective proteins and cause damage to the mitochondrial DNA.

The proper functioning of the mitochondria requires a sufficient amount of all these proteins as well as their integrity. An active capable of positively regulating the production of these key proteins is considered as a tool against skin aging. The following proteins and their functions can be mentioned: MPV17: involved in the maintenance of the mitochondrial genome and the regulation of the metabolism of reactive oxygen species.

DNJB4: involved in the response to heat and protein folding.

SELT: involved in redox balance and antioxidant action.

TIM14: action on protein transport inside the inner membrane of the mitochondria (participates in the protein complex allowing this transport).

MFN2: involved inter alia in the organization of the mitochondrial membrane.

PTCD3: involved in the mechanisms of mitochondrial translation.

MTCH1: involved in the regulation of the apoptotic process.

TIMMDC1: involved in the assembly of complex I of the respiratory chain.

TOM20: involved in the assembly of the complex allowing translocation in the outer mitochondrial membrane.

SLC25A20: involved in transport within the mitochondria.

hPREP: a protease that destroys a marker peptide that allows protein entry into the mitochondria, after this peptide has detached from the protein.

MsRA: detoxifying action on oxidized proteins.

TIM16: action on the transport of proteins inside the inner membrane of the mitochondria (participates in the protein complex allowing this transport).

MFTC: transports vitamin folate in the mitochondria.

LYRM7: assembly factor of complex III essential for homeostasis of mitochondria.

### Principe:

HDFs are grown until a confluent mat is obtained. They were then brought into contact with 10 ppm of the mixture of cyclic peptides according to the invention (or its excipient for the control cases) for 10 days, with change of the medium every 3 days. At the end of this contact, the cells were lysed so as to extract the proteins and to analyze them in the form of crushed material by a method associating the action of a protease on the crushed material, the separation of the fragments by coupled liquid chromatography to mass spectrometry then the identification and concentration of pre-existing proteins according to the nature and the quantity of the fragments obtained. An analysis of variance and a Student t-test for non-paired series were performed to evaluate the significance of the results.

N = 3 for each condition.

### Results:

**Table 8: Variation versus control of different mitochondrial proteins of fibroblasts. Effect of 10 ppm of the mixture of cyclic peptides according to the invention.**

| **Protein** | **MPV17** | **DNJB4** | **SELT** | **TIM14** | **MFN2** | **PTCD3** |
|---|---|---|---|---|---|---|
| **Variation** | x 2.08 | x 2.39 | x 1.76 | x 1.74 | x 1.69 | x 1.58 |
| | *p<0.01* | *p<0.01* | *p<0.05* | *p<0.01* | *p<0.01* | *p<0.01* |

| **Protein** | **MTCH1** | **TIMMDC1** | **TOM20** | **SLC25A20** | | |
|---|---|---|---|---|---|---|
| **Variation** | x 2.64 | x 2.73 | x 3.52 | x 6.43 | | |
| | *p<0.01* | *p<0.01* | *p<0.01* | *p<0.01* | | |

These results show that 10 ppm of the mixture of cyclic peptides according to the invention are able to stimulate the synthesis of several mitochondrial proteins involved in the transport of proteins in the mitochondria, the defense of mitochondria facing oxidative stress, dynamism and of mitochondria homeostasis. These stimulations improve the metabolism of the mitochondria and, by the same, cutaneous cells containing them.

### 2/ Comparative tests: purified cyclic peptides versus cyclic peptide mixture

The aim of this part is to compare the activity of the purified cyclic peptides according to the invention with that of a mixture of cyclic peptides according to the invention. The tests used for this comparison are those relating to the synthesis of collagen and elastin by fibroblasts.

### 2.1/ Collagen I and elastin synthesis by ELISA

### Principe:

HNFs were grown in 24-well plates for 24 hours. The cells were placed in contact or not with test products at different concentrations for 3 days. The collagen synthesis was evaluated by ELISA assay on the culture supernatant and the result was reduced to the number of cells.

### Results:

**Table 9: Comparison of the collagen I production between the mixture of cyclic peptides according to the invention and two purified cyclic peptides (ELISA method).**

| **Concentrations** | **Linusorb B3** | **Linusorb B2** | **Mixture of cyclic peptides** |
|---|---|---|---|
| **10 ppm** | *-0.4%; nds* | +36.2%; *p<0.01* | +60.2%; *p<0.01* |
| **15 ppm** | +62.6%; *p<0.01* | +39.2%; *p<0.01* | +121.6%; *p*<*0.01* |

**Table 10: Comparison of the production of elastin between the mixture of cyclic peptides according to the invention and two purifified cyclic peptides (ELISA method).**

| **Concentration** | **Linusorb B3** | **Linusorb B2** | **Mixture of cyclic peptides** |
|---|---|---|---|
| **12.5 ppm** | +58%; *p<0.05* | +43%; *p<0.05* | +72%; *p*<*0.01* |

### 2.2/ Collagen synthesis by IMF

### Principe:

FHNs were grown in 24-well plates for 24 hours. The cells were placed in contact with test products (or their excipient) at different concentrations for 6 days. The collagen I produced by the cells was then quantified by immunofluorescence on the fixed mats, and the result was then reduced to the number of cells.

### Results:

**Table 11: comparison of the production of collagen I between the mixture of cyclic peptides according to the invention and two purified cyclic peptides (IMF method).**

| **Concentrations** | **Linusorb B3** | **Linusorb B2** | **Mixture of cyclic peptides** |
|---|---|---|---|
| **5 ppm** | +111.1; *p*<*0.01* | +34.2; *dns* | +118.2; *p<0.01* |
| **10 ppm** | +121.7; *p*<*0.01* | +41.8; *p*<*0.01* | +149; *p*<*0.01* |

The results of these tests show the advantage of using a mixture of cyclic peptides in certain cases with respect to purified cyclic peptides.

### 3/ Association of the mixture of cyclic peptides according to the invention with a seed extract of Apium graveolens

### Principe:

The principes in the different tests presented below are the same as those described previously in this part, in point 1/. In these tests, it is the active ingredient according to the invention defined above in B) 2), associating 500 ppm of mixture of cyclic peptides (see part A) and 700 ppm of the supercritical CO₂ extract of seeds of *Apium graveolens* (at the end comprising 0.05% of total phthalides), as described in part B), which was evaluated. This active ingredient was tested at 1.5%, 2% or 3%; that is to say respectively 7.5 ppm, 10 ppm or 15 ppm of the cyclic peptide mixture, and 10.5 ppm, 14 ppm or 21 ppm of the supercritical CO₂ extract of *Apium graveolens* seeds.

For the *in vitro* tests, cyclic peptides and alkyl phthalides were pre-solubilized in acid DMSO (for cyclic peptides) and in ethanol (for the *Apium graveolens* seed extract containing alkyl phthalides), before to be introduced in the medium of each test. Therefore, reference is thereafter made to an equivalent of the active ingredient (in Tables 13-16 and 18 below).

### Results:

**Table 12:**

| **Equivalent of the active ingredient according to the invention in the test** | **Concentration of cyclic peptides according to the invention** | **Concentration of the CO₂ extract of *Apium graveolens* seeds** |
|---|---|---|
| 1.5% | 7.5 ppm | 10.5 ppm |
| 2% | 10 ppm | 14 ppm |
| 3% | 15 ppm | 21 ppm |

### 3.1/ Decrease of inflammation mediators

**Principe:** as disclosed above at point 1.3/.

### Results:

**Table 13: Variation of the amount of PGE₂ and IL-6 produced by fibroblasts after contact with different % of the equivalent of the active ingredient according to the invention.**

| | **IL-6 % of variation / control** | **PGE-2 % of variation / control** |
|---|---|---|
| **Non-irradiated control (NI)** | **Reference** | **Reference** |
| **Active: 2% (NI)** | -36; *p<0.01* | -38; *p<0.05* |
| **Active: 3% (NI)** | -56; *p<0.01* | -36; *p<0.05* |
| **Control (UVB)** | +42 | +435 |
| | Reference UVB | Reference UVB |
| **Active: 2% (UVB)** | -49; *p*<*0.01* | -51; *p<0.01* |
| **Active: 3% (UVB)** | -70; *p*<*0.01* | -67; *p*<*0.01* |

These data show that the active ingredient according to the invention significantly moderates the basal productions, in the absence of irradiation, of IL6 and PGE2 mediators known for their involvement in micro-inflammations.

As expected, the stress model used (UVB) induces large increases in IL-6 and PGE2 in the control without active. The active ingredient according to the invention reduces these inductions in a dose-dependent and significant manner.

These results were confirmed on keratinocytes in complementary tests in which the active ingredient according to the invention reduces, in a dose-dependent manner, the production of UVB-induced IL-6 (-48%, *p<0.05,* at 3% of its equivalent).

### 3.2/ Reduction of sebum production by sebocytes

**Principe:** as disclosed at point 1.4/ above.

### Results:

**Table 14: Modulation of lipid synthesis in sebocytes after contact with different concentrations of the equivalent of the active ingredient according to the invention; (n=3)**

| | **Intracellular lipids % of variation / control** |
|---|---|
| **Control solvent** | Reference |
| Actif: 2% | -54; *p<0.01* |
| Actif: 3% | -45; *p<0.01* |

As in the previous studies, there is a clear reduction in lipid storage in sebocytes *in-vitro.* This lower storage (-54% for the equivalent of 2% of the active ingredient according to the invention) compared to the control cases indicates a lower production of lipids forming sebum.

### 3.3/ Cross-talking (tests with conditioned media)

The same tests as those described in point 1.5/ above were conducted and demonstrated that the mixture of cyclic peptides and *Apium graveoloens* seed extract according to the invention also had the capacity to reduce the production of stratifin by keratinocytes subjected to UVB irradiation (-62% at 10ppm; *p*>*0.01*) and consequently greatly limit the effects induced by this protein on fibroblasts (collagen synthesis decreases and protein proteases increase in the dermis).

### 3.4/ Action on mitochondrial proteins

**Principe:** as disclosed above at point 1.6/.

### Results:

**Table 15: Variation vs. control of different mitochondrial proteins of fibroblasts. Effect of 2% of the equivalent of the active ingredient according to the invention.**

| **Concentration** | **MPV17** | **hPREP** | **MsRA** | **TIM14** | **TIM 16** | **TOM20** | **MFTC** | **LYRM7** |
|---|---|---|---|---|---|---|---|---|
| **Active: 2%** | x 3.47* | x 1.51* | x 3.32** | x 3.74* | x 2.09** | x 3.64* | x 4.28* | x 3.59* |

| | | | | | | | | |
|---|---|---|---|---|---|---|---|---|
| **p<0.01; **p<0.05.* | | | | | | | | |

These results show that the active ingredient according to the invention stimulates the synthesis of several proteins involved in the transport of proteins in the mitochondria or in the defense of the mitochondria. These stimulations will improve the mitochondrial metabolism.

### 3.5/ Action on maintaining skin barrier integrity

The *stratum corneum* is the outermost layer of the epidermis. It is continually renewed by desquamation. It forms a hydrophobic barrier that is very effective with respect to the molecules of the external environment. In addition, it limits the loss of water of the body, thanks in particular to the NMF. This barrier is an assembly of great complexity associating, on the one hand, flat cells without nucleus, strongly linked, and on the other hand, lipids and proteins whose composition and assembly ensure the unique properties of this structure very resistant to physical, chemical and biological attacks of the environment. It is therefore important in a perspective of maintaining a good hydration of the skin to keep the skin barrier in good condition.

The following tests show that the ingredient according to the invention makes it possible to meet this aim: the first test at a visual level (state of differentiation of a cell layer), the other tests at a molecular level by assaying different markers of epidermal differentiation on two distinct biological systems (human keratinocytes thin layer culture or human skin explants culture).

### a) Improvement of keratinocyte differentiation quality

Near-confluent human keratinocytes were contacted 2, 3 and 6 days with the equivalent of the active ingredient according to the invention (or its placebo) in a suitable culture medium in order to study their phenotypic modifications under the microscope, according to the appearance of the cells. It is observed with the equivalent of the active ingredient according to the invention (from 1%) a clear acceleration of the phenotypes that are characteristic of the differentiation with the presence of typical structures of the upper layers of the epidermis (presence of branched structures characteristic of the proteolipidic rigid matrix and in multilayer of the horny envelope; refractive network).

### b) Study of keratinocyte differentiation

### Principe:

The same cell layers as those studied above in fresh state in the part are stopped after 4 days of contact with the equivalent of the active ingredient according to the invention (or its solvent control). They were then fixed and immunostained (through the use of specific primary antibodies and fluorescent secondary antibodies) to visualize the synthesis of 2 epidermal differentiation markers, loricrin and involucrine. 5 photos were made on each of the 3 replicas. Quantification of the markings was performed by image analysis. Counterstaining the cell nuclei makes it possible to estimate the number of cells and thus normalize the data. An analysis of variance and a non-paired student test t were performed to validate the significance of the results.

### Results:

**Table 16: Modulation of the expression of loricrine and involucrine by keratinocytes in contact with the equivalent of the active ingredient according to the invention at 2%.**

| | **Loricrine % of variation / control** | **Involucrine % of variation / control** |
|---|---|---|
| **Control** | Reference | Reference |
| **Active: 2%** | +222%; *p*<*0.01* | +56%; *p<0.01* |

### c) Study of the differentiation on explants

### Principe:

Standardized abdominal skin explants from a patient were received immediately after collection. After acclimation to 37°C, a cream containing 3% of the active ingredient according to the invention or a placebo cream is applied to the surface of the explants 1 time per day for 6 days with a daily renewal of the application. After 6 days of culture the explants were stopped, frozen in nitrogen and cut. Immunolabeling of the differentiating marker proteins PNPLA1, K24 and K10 was then carried out by the use of specific primary antibodies and fluorescent secondary antibodies. Each condition was photographed under a fluorescence microscope. Quantification of the markings was performed by image analysis. An analysis of variance and a non-paired student test t are performed to validate the significance of the results.

### Results:

**Table 17: Modulation of the expression of PNPLA1, K24 and K10 on skin explants. Effect of the active ingredient according to the invention at 3%.**

| | **PNPLA1 % of variation/control** | **K24 % of variation/control** | **K10 % of variation/control** |
|---|---|---|---|
| **Control** | Reference | Reference | Reference |
| **Active: 3%** | +16; *p<0.01* | +44; *p<0.05* | +39; *p*<*0.01* |

### d) Synthesis of neutral lipids on keratinocytes

### Principe:

The same cell layers as those studied above in the fresh state were stopped after 4 days of contact with 2% of the equivalent of the active ingredient according to the invention (or its placebo). They were then fixed and stained with Nile Blue (neutral lipid markers) and a nuclei marker for cell quantification. Photos were taken to evaluate each marking and quantified by image analysis. An analysis of variance and a non-paired student test t were performed to validate the significance of the results.

### Results:

**Table 18: Modulation of the neutral lipid synthesis by keratinocytes in contact with the equivalent of the active ingredient according to the invention at 2%.**

| | **% of variation/control** |
|---|---|
| **Control solvent** | Reference |
| **Active: 2%** | +92; *p*<*0.01* |

All these results agree to show the interest of the active ingredient according to the invention in the establishment, reinforcement and restoration of the cutaneous barrier. The known protein markers of the quality of the formation of this barrier: involucrine, loricrine PNPLA1, K24 and K10 are all increasing thanks to the active ingredient according to the invention. It is the same for a category of lipids that is determining for the formation of the *stratum corneum:* the neutral lipids.

### D) GALENIC

Various compositions/formulations are described below. Additional active cosmetic ingredients, optionally supporting and/or complementing the activity of the active ingredient according to the invention may be added in the appropriate phase according to their hydrophobic or hydrophilic nature. These ingredients can be of any category depending on their function(s), the place of application (body, face, neck, bust, hands, hair, eyelashes, eyebrows, hair, etc.), the desired end effect and the targeted consumer, for example antioxidant, tensor, moisturizer, nourishing, protective, smoothing, remodeling, volumizing (lipofiling), acting on the radiance of the complexion, against undereye bags and dark circles, concealer, anti-glycation, slimming, soothing, myo-relaxing, anti-redness, anti-stretch marks, sunscreen, etc. They are mentioned above in the description.

Active ingredient according to the invention: as described at the point B.1/ above or B.2/ above (further comprising an extract of *Apium graveolens* seeds). This ingredient can be formulated between 1 and 5%, preferably 3%.

### 1) Cream form, for example an antiaging day cream for the face.

| **Raw materials** | **INCI name** | **Role** | **%** |
|---|---|---|---|
| Part A: | | | |
| . H₂O | / | / | qsp100 |
| . Carbopol^{™} Ultrez 10 | . Carbomer | . Thickening/gelling agent | 0.30 |
| Part B: | | | |
| . Brij S2-SS-(RB)^{™} | . Steareth-2 | . Emulsifier | 0.40 |
| . Brij S10-SO-(RB)^{™} | . Steareth-10 | . Emulsifier | 1.20 |
| . Crodafos CES-PA-(RB)^{™} | . Cetearyl Alcohol (and) Dicetyl Phosphate (and) Ceteth-10 Phosphate | . Emulsifier /conditionner | 4.00 |
| . Crodamol ^{™} OSU-LQ-(JP) | . Diethylhexyl Succinate | . Emollient | 4.00 |
| . Crodamol ^{™} AB-LQ-(RB) | . C12-15 Alkyl Benzoate | . Emollient | 1.50 |
| Part C: | | | |
| . Ingredient according to the invention | / | . Active | 3.00 |
| Part D: | | | |
| . Glycerin | . Glycerin | . Humectant | 4.00 |
| . Octanediol | . Caprylyl Glycol | . Humectant/ Emollient | 0.50 |
| Part E: | | | |
| . Phenoxyethanol | . Phenoxyethanol | . Preservative | qs |
| Part F: | | | |
| . Potassium sorbate | . Potassium Sorbate | . Preservative | qs |
| Part G: | | | |
| . H₂O | / | / | 4.00 |
| . NaOH 30 % | . Sodium Hydroxide | . pH adjuster | 0.40 |

### Example(s) of additional ingredient(s):

- A sebo-regulator ingredient such as:
   **SEBULESS^{™}:** sold by Sederma, comprising an extract of *Syringa vulgaris* obtained by *in vitro* cell culture, which is a sebum regulator, purifying, mattifying and refreshing the complexion and blurrying imperfections.
      **Ac-Net^{™}:** sold by Sederma for a treatment of oily and acne-prone skins.
   **EVERMAT^{™}:** sold by Sederma, comprising a combination of an *Enantia chlorantha* extract rich in protoberberines and oleanolic acid; it decreases pore size and brightness; refines the grain of acne-prone skins.
- A tensing ingredient such as:
   **IDEALIFT^{™}:** sold by Sederma, comprising the lipodipeptide N-acetyl-Tyrosyl-Arginyl-O-hexadecyl ester, combating the flaccidity of the face and improving the resistance to gravity, *via* stimulation of elastin in particular.

**2) Fluid form cream,** for example to realise a light firming base for the face for use before makeup (acting in particular on the densification of the dermis and thus on the tightening of the pores).

| **Raw materials** | **INCI name** | **Role** | **%** |
|---|---|---|---|
| Part A: | | | |
| . H₂O | / | / | qsp100 |
| . Pentylen glycol | . Pentylene glycol | . Humectant | 3.00 |
| . Phenoxyethanol | . Phenoxyethanol | . Preservative | 0.80 |

| Part B: | | | |
|---|---|---|---|
| . Ingredient according to the invention | / | . Active | 3.00 |
| . Crodamol^{™} GTEH | . Triethylhexanoin | . Emollient | 1.00 |
| . VisiaOptima^{™} SE | . Sodium Polyacrylate (and) Ethylhexyl Cocoate (and) PPG-3 Benzyl Ether Myrisate (and) Polysorbate 20 | . Rheology mofifier and emulsion stabilizer | 0.80 |

| Part C: | | | |
|---|---|---|---|
| . Potassium sorbate | Potassium Sorbate | . Preservative | 0.10 |

### Example(s) of additional ingredient(s):

- A soothing ingredient for sensitive skin such as:
   **Phytofleur^{™} Lily,** sold by Crodarom, comprising an extract of flowers of *Lilium candidum.*
- A moisterizing ingredient such as:
   **AQUALANCE^{™}**, sold by Sederma, osmoprotective compound composed of homarine and erythritol.
- An antiaging ingredient such as:
   **RESISTEM^{™},** sold by Sederma, helping skin to build its own anti-aging defense system, based on an extract obtained by *in-vitro* cell culture of the plant *Globularia cordifolia.*

### 3) Mask form

| **Raw materials** | **INCI name** | **Role** | **%** |
|---|---|---|---|
| Part A: | | | |
| . H₂O | / | / | qsp100 |
| . Potassium sorbate | . Potassium sorbate | . Preservative | 0.10 |
| . Glycerine | . Glycerin | . Humectant | 10.00 |
| . Volarest^{™} FL | . Acrylates/Beheneth-25 Methacrylate Copolymer | . Rheology modifier | 1.00 |

| Part B: | | | |
|---|---|---|---|
| . Ingredient according to the invention | / | . Active | 3.00 |
| . Crodamol^{™} ISIS | . Isostearyl Isostearate | . Emollient | 3.00 |
| . Cromollient^{™} DP3A | . Di-PPG-3 Myristyl Ether Adipate | . Emollient | 2.00 |
| . ViscOptima^{™} SE | . Sodium Polyacrylate (and) Ehtylhexyl Cocoate (and) PPG-3 | Rheology modifier and | 2.50 |
| | Benzyl Ether Myristate (and) Polysorbate 20 | emulsion stabilizer | |
| . Phenoxyethanol | . Phenoxyethanol | . Preservative | 0.80 |

| Part C: | | | |
|---|---|---|---|
| . Tween^{™} 20 | . Polysorbate 20 | . Emulsifier | 0.50 |

| Partie D : | | | |
|---|---|---|---|
| . H₂O | / | / | 4.00 |
| . Sodium hydroxyde 30% | . Sodium hydroxyde | . pH adjuster | 0.40 |

### Example(s) of additional ingredient(s):

- an ingredient with a "refreshing" effect such as:
   **Covafresh^{™}** (Mentyl Lactate (and) PPG-26-Buteth-26 (and) PEG-40 Hyrogenated castor Oil), sold by Firmenich.
- a soothing ingredient for sensitive skin such as:
   **Pacifeel^{™}**, sold by Sederma, comprising an extract of *Mirabilis Jalapa.*
- a tensor/soother ingredient such as:
   **Dynalift^{™}**, sold by Sederma, comprising an extrat of Sorgho juice rich in polyosides and sucroses.
   **Tenseur vegetal ST,** sold by Sederma, comprising a polymannuronate (an hydrophylic polysaccharide of *Macrocystis pyrifera* algae) and prolamines (wheat proteins having a high molecular weight and being very rich in glutamine/glutamic acid).

### 4) Light texture form evaporating to leave only a veil on the skin

| **Raw materials** | **INCI name** | **Role** | **%** |
|---|---|---|---|
| Part A: | | | |
| . H₂O | / | / | qsp100 |
| . Potassium sorbate | . Potassium sorbate | . Preservative | 0.10 |

| Part B: | | | |
|---|---|---|---|
| . Glycerin | . Glycerin | . Humectant | 5.00 |
| . Phenoxyethanol | . Phenoxyethanol | . Preservative | 0.80 |
| . Keltrol^{®} CG-SFT | . Xanthan Gum | . Rheology modifier | 1.00 |

| Part C: | | | |
|---|---|---|---|
| . Crodafos^{™} MCK | . Potassium Cetyl Phospate | . Emulsifier | 4.00 |

| Part D: | | | |
|---|---|---|---|
| . Duraquench^{™} IQ SA | . Cetyl Stearate (and) Isostearyl Isostearate (and) Cetyl Alcohol (and) Potassium Cetyl Phosphate (and) Stearic Acid | . Mosturizing agent | 1.00 |
| . Arlamol^{™} HD | . Isohexadecane | . Emollient | 3.00 |
| . Crodamol^{™} AB | . C12-15 Alkyl Bezoate | . Emollient | 1.50 |
| . Crodamol^{™} STS | . PPG-3 Benzyl Ehter Myristate | . Emollient | 1.00 |
| . Ingredient according to the invention | | . Active | 3.00 |

| Part E: | | | |
|---|---|---|---|
| . H₂O | / | / | 6.00 |
| . Lactic acid | . Lactic Acid | . pH adjuster | 0.60 |

### Example(s) of additional ingredient(s):

- an antioxidant/anti-aging ingredient such as:
   **MAJESTEM^{™},** sold by Sederma, based on plant cells of *Leontopodium alpinum* obtained by *in-vitro* cellular culture and being titrated in leontopodic acid; neutralises oxidative stress (pollution, UV radiations) and restores cutaneous tension.
   **SENESTEM^{™},** sold by Sederma, comprising plant cells obtained by *in-vitro* cellular culture of *Plantago lanceolata.*
- A moisterizing ingredient such as:
   **Crodarom^{®} Honey,** sold by Crodarom, based on honey.

### 5) Oily cream form, for example for making a night mask

| **Raw materials** | **INCI name** | **Role** | **%** |
|---|---|---|---|
| Part A: | | | |
| . H₂O | / | / | qsp100 |
| . Volarest^{™} FL | . Acrylates/Beheneth-25 Methacrylate Copolymer | . Rheology modifier | 2.50 |
| . Potassium sorbate | . Potassium Sorbate | . Preservative | 0.10 |
| . Sodium sulfite | . Sodium Sulfite | . Antioxydant | 0.01 |

| Part B: | | | |
|---|---|---|---|
| . Arlatel^{™} LC | . Sorbitan Stearate (and) Sorbityl Laurate | . Emulsifier | 1.70 |
| . Glycerin | . Glycerin | . Humectant | 5.00 |
| . Propanediol | . Xanthan Gum | . Humectant | 5.00 |
| . Phenoxyethanol | . Phenoxyethanol | . Preservative | 0.80 |
| . Tween^{™} 20 | . Polysorbate 20 | . Emulsifier | 1.00 |

| Part C: | | | |
|---|---|---|---|
| . Crodamol^{™} ISIS | . Isostearyl Isostearate | . Emollient | 3.00 |
| . Crodamol^{™} GTIS | . Triisostearin | . Emollient | 2.00 |
| . Crodamol^{™} AB | . C12-15 Alkyl Benzoate | . Emollient | 1.50 |
| . Cromollient^{™} DP3A | . Di-PPG-3 Myristyl Ether Adipate | . Emollient | 1.00 |
| . Crodamol^{™} SSA | . Decyl Isostearate (and) Isostearyl Isostearate | . Emollient | 1.00 |
| . Ingredient according to the invention | / | . Active | 3.00 |
| . Tween^{™} 60 | . Polysorbate 60 | . Emulsifier | 1.00 |

| Part D: | | | |
|---|---|---|---|
| . H₂O | / | / | 2.00 |
| . Sodium hydroxyde 30% | . Sodium Hydroxyde | . pH adjuster | 0.20 |

### Example(s) of additional ingredient(s):

- A moisterizing/soothing ingredient such as:
   **Optim Hyal^{™},** sold by Sederma, containing oligosaccharides of acetylated glucuronic acids having a structure analogue to fragments of hyaluronic acid.
- An ingredient acting on undereye bags and dark circles such as:
   **HALOXYL^{™},** sold by Sederma, combining 2 matrikines, the Pal-GHK and the Pal-GQPR with N-hydroxysuccinimide and a flavonoid, the chrysine.
   **EYELISS^{™}**, sold by Sederma, combining 3 components the hesperidine methyl chalcone, the dipeptide Valyl-Tryptophan (VW) and the lipopeptide Pal-GQPR.
   **PRODIZIA^{™}**, sold by Sederma, comprising an extract of *Albizia julibrissin,* which helps reduction of the visible signs of fatigue: dark circles, undereye bags, dull complexion and drawn facial features, by reparing and protecting skin from the damages of glycation.

### 6) Blur emulsion form, light diffusing

| **Raw materials** | **INCI name** | **Role** | **%** |
|---|---|---|---|
| Part A: | | | |
| . Microcare^{(R)} Silicone W3045 | . C30-45 Alkyl Dimethicone | . O/W co-emulsifier | 2.00 |
| . Microcare^{(R)} Silicone E9016 | . Cetyl PEG/PPG-10/1 Dimethicone | . O/W emulsifier | 2.00 |
| . Microcare^{(R)} Silicone M1600 | . Cetyl Dimethicone | . Emulsion stabilizer | 5.00 |
| . Arlamol^{™} HD | . Isohexadecane | . Emollient | 4.00 |
| . Xiameter^{®} PMX 200 5cs | . Dimethicone | . Silicon oil | 2.00 |
| . Covabead Velvet 10 | . Polymethyl Methacrylate | . Light focus polymer | 2.00 |
| . KSG-016F | . Dimethicone (and) Dimethicone/Vinyl Dimethicone Crosspolymer | . Silicon elastomer having a matifying effect | 12.00 |
| . SH219 | . Silica (and) Titanium Dioxide | . light focus powder | 6.00 |
| . Coviox^{®} T70 | . DI Alpha Tocopherol | . Antioxydant | 0.10 |
| . Ingredient according to the invention | / | . Active | 3.00 |

| Part B: | | | |
|---|---|---|---|
| . H₂O | / | / | qsp 100 |
| . Sodium chlorure | . Sodium Chlorure | . Stabilizer | 1.00 |
| . Potassium sorbate | . Potassium Sorbate | . Preservative | 0.10 |

| Part C: | | | |
|---|---|---|---|
| . Glycerin | . Glycerin | . Humectant | 5.00 |
| . Phenoxyethanol | . Phenoxyethanol | . Conservateur | 0.80 |

### Example(s) of additional ingredient(s):

- A global antiageing ingredient such as:
   **RENOVAGE^{™}**, sold by Sederma, based on teprenone.

### 7) Stick form, for example for a localized effect on the "T" area of the face

| **Raw materials** | **INCI name** | **Role** | **%** |
|---|---|---|---|
| Part A: | | | |
| . Crodacol^{™} C90 | . Cetyl Alcohol | . Structuring agent | 24.00 |
| . Syncrowax^{™} HRC | . Tribehenin | . Structuring agent | 7.00 |
| . Span^{™} 60 | . Sorbitan Stearate | . Emulsion stabilizer | 2.00 |
| . Ingredient according to the invention | / | . Active | 3.00 |
| Part B: | | | |
| . Butylen glycol | . Butylene Glycol | . Humectant | qsp 100 |
| . Phenoxyethanol | . Phenoxyethanol | . Preservative | 0.80 |
| . Crodesta^{™} F160 | . Sucrose Stearate | . Emulsifier | 3.00 |

### Examples of additional ingredients:

- A sunscreen ingredient such as:
   **Solaveil^{™} CT-300** (Titanium Dioxide (and) Caprylic/Capric Trigyceride (and) Polyhydroxystearic Acid (and) Aluminium Stearate (and) Alumina), sold by Croda.
- A revitalizing ingredient such as:
   **Floral Nectar^{™},** sold by Crodarom, an extract of the flowers of *Combretum farinosum.*
- An antipollution ingredient such as:
   **CITYSTEM^{™},** sold by Sederma, based on plant cells obtained *in-vitro* of *Marrubium vulgare* having a high concentration of Forsythoside B; used against pollution attacks: skin is soother and smoother, skin grain is refined, the visibility of comedones is attenuated, leaving the skin radiant and purified.

### 8) Transparent oily balm form

| **Raw materials** | **INCI name** | **Role** | **%** |
|---|---|---|---|
| Part A: | | | |
| . Oleocraft^{™} LP-20 | . Polyamide 8 | . Structuring oily polymer | 25.00 |
| . Crodamol^{™} AB | . C12-15 Alkyl Benzoate | . Light emollient | Qsp 100 |
| . Prisorine^{™} 3515 | . Isostearyl Alcool | . Cleansing emollient | 7.00 |
| . Arlasolve^{™} DMI PC | . Dimethyl Isosorbide | . Emollient | 4.00 |
| . Pentylene Glycol | . Pentylene Glycol | . Emollient | 3.00 |
| . Crodamol^{™} OP | . Ethylhexyl Palmitate | . Emollient | 10.00 |
| . Arlamol^{™} HD | . Isohexadecane | . Emollient | 3.00 |
| . DI Alpha Tocopherol | | . Antioxidant | 0.50 |
| . Ingredient according to the invention | / | . Active | 3.00 |
| Part B: | | | |
| . Span^{™} 120 | . Sorbitan Isostearate | . Emulsifier | 3.00 |
| . Cithrol^{™} 10GTIS | . PEG-20 Glyceryl Triisostearate | . Emulsifier | 15.00 |
| . H₂O | / | / | 3.00 |
| Part C: | | | |
| . Phenoxyethanol | . Phenoxyethanol | . Preservative | 0.40 |

### 9) Tonic form

| **Raw materials** | **INCI name** | **Role** | **%** |
|---|---|---|---|
| Part A: | | | |
| . H₂O | / | / | Qsp 100 |
| . Potassium sorbate | . Potassium Sorbate | . Preservative | 0.10 |
| Part B: | | | |
| . Croduret^{™} 54 | . PEG-54 Hydrogenated Castor Oil | . Surfactant | 4.50 |
| . Tween^{™} 60 | . Polysorbate 60 | . Solubilizer | 3.00 |
| . Cromollient^{™} SCE | . Di-PPG-2 Myteth-10 Adipate | . Emollient | 1.00 |
| . Ingredient according to the invention | / | . Active | 3.00 |
| Part C: | | | |
| . Propanediol | . Propanediol | . Humectant | 5.00 |
| . Phenoxyethanol | . Phenoxyethanol | . Preservative | 0.80 |

### Example(s) of additional ingredient(s):

- A tonic ingredient such as:
   **CHRONODYN^{™}**, sold by Sederma, which is a biotechnological extract of *Euglena gracilis,* tonifies and strengthens the skin, erases fatigue marks.

### E) In-vivo EFFICACY TESTS

**Tested product:** the cream 1) of point D) of the Galenic above.

**Principes:** the evaluation of the efficacy of the mixture was conducted on a total of 31 volunteers in a study, against placebo, to measure the improvement of the quality of the skin by following the following parameters: re-densification, firmness, state surface and re-pulping effect.

Several complementary techniques have been used to study different parameters:
- an evaluation of visco-elastic properties, performed with a cutometer;
- an evaluation of the dermal density, carried out with a Translucymeter;
- an evaluation of the pore support tissues by confocal laser microscopy; and
- an evaluation of the surface texture performed with a Goniolux^{®} and a silicone impression analysis.

### Protocol:

### Specific inclusion criteria

The study was conducted on a panel of 31 women volunteers, mean age 47 years (35 - 58 years), visually presenting a decrease in densification of the skin found by the presence of slightly dilated pores. They were asked for a hormonal consistency during the 3 months preceding the test and during the test. In addition, the volunteers had to observe a wash-out period of 10 days, with a moisturizer and exclude any cosmetic act of scrub or mask type.

### Type of study, duration, applications

The volunteers were not aware of the composition of the two products tested, these being organoleptically and visually identical. The creams were applied to the face, each volunteer applied on one side of the face a cream according to the invention and a placebo cream on the other side of the face. The creams were applied in bi-daily massage for 8 weeks.

### The synopsis of the study can be summarized according to the below diagram below

| T0 | T4weeks | T8weeks |
|---|---|---|
| -----------------------------------------------------------------------------------------------------------------------------------→ | | |
| Texture (Goniolux^{®}/Prints) | Texture (Goniolux^{®}/Prints) | |
| Cutometer | | Cutometer |
| Translucymeter | | Translucymeter |
| Confocal laser microscopy | | Confocal laser microscopy |

### Statistics

Statistical studies were performed using Student t-test or, if required, a non-parametric Wilcoxon test. The tests were performed on matched series.

### Method and results

### Evaluation of the viscoelastic properties of the cheek

The Cutometer^{®} MPA580 (Courage & Khazaka) was used to measure viscoelastic parameters on the cheek of volunteers. This device measures the deformation of a cutaneous zone, subjected to repeated mechanical stresses of suction, as well as its recovery power. The apparatus provides graphs of deformation of the skin as a function of time as shown in FIG. 3. In this figure are indicated the parameters Ur / Ue, Ur / Uf and Ur, respectively called Firmness, Elasticity and Tone, varying with age, that were chosen. Three independent acquisitions were made at T0 and T8 weeks.

### Results:

**Table 19: Variation of firmness, elasticity and tone of the cheek - effect of the mixture of the invention and placebo. (n = 30 vol.; n = 3 measures)**

| **Cutometer^{®}** | **Placebo** | | | | | |
|---|---|---|---|---|---|---|
| | **Firmness** | | **Elasticity** | | **Tone** | |
| | Ur/Ue | | Ur/Uf | | Ur | |
| | T0 | T8s | T0 | T8s | T0 | T8s |
| **Mean +/- standard deviation** | 0.394 +/- 0.059 | 0.399 +/- 0.071 | 0.275 +/- 0.044 | 0.277 +/- 0.037 | 0.0982 +/- 0.0166 | 0.0975 +/- 0.0176 |
| **% variation *vs*. T0** | | +1.3% | | +0.7% | | -0.7% |
| Significance | | *nsd* | | *nsd* | | *nsd* |

| **Cutometer^{®}** | **Cream according to the invention** | | | | | |
|---|---|---|---|---|---|---|
| | **Firmness** | | **Elasticity** | | **Tone** | |
| | Ur/Ue | | Ur/Uf | | Ur | |
| | T0 | T8s | T0 | T8s | T0 | T8s |
| **Mean +/- standard deviation** | 0.388 +/-0.069 | 0.420 +/-0.065 | 0.269 +/-0.041 | 0.288 +/- 0.038 | 0.0961 +/-0.0162 | 0.1005 +/-0.0131 |
| **% variation *vs*.T0** | | **+8.2%** | | **+7.1%** | | **+4.6%** |
| Significance | | ***p<0.05*** | | ***p<0.05*** | | ***p<0.05*** |
| Maximum | | **55%** | | **47%** | | **35%** |
| Responders | | **77%** | | **73%** | | **77%** |
| Significance vs. placebo | ***p<0.05*** | ***p<0.05*** | ***p<0.05*** | | | |

The results in the table above show a net increase of the three parameters studied.

These results therefore show a clear improvement of the viscoelastic parameters of the cheek related to the re-pulping effect of the cream according to the invention, firmness + 8.2%; Elasticity + 7.1 % and Tone + 4.6%. This improvement is significant (*p <0.05*) compared with placebo. These results are in line with those obtained *in-vitro* showing that cyclic peptides activate collagen-I synthesis and moderate the "cross-talking" of the anti-matrix agents.

### Evaluation of the dermal density of the cheek

### Principe:

As explained above, skin density as part of anti-aging strategies is very important. The quality of the skin has been evaluated for a long time non-invasively using sound measurements (ultrasound for example) or through different types of lights (UV, visible, IR) which allows measurements to be made on and in the skin. A Translucymeter^{®} TLS 850 (Diastron), measuring the path of light in the skin, was used. The skin, like many materials, is neither totally transparent (transmission of a clear image), nor totally opaque (no transmitted light): it is translucent. The degree of translucency depends on the absorption coefficient and dispersion of the material with regards to the light entering it.

Translucymeter^{®} illuminates the skin with narrow beams of color. These lights penetrate into the skin where they meet the layers (junctions between *stratum corneum* and epidermis, epidermis-dermis ...) and macromolecules (especially collagen fibers which represent 80% of the mass of the skin). These fibers are of very different density and quality depending on depth and age. The lights will be more or less absorbed or deflected in different directions or returned to the probe (backscattering), the latter analyzing the signal and translating it into light level.

### Results:

Measurements at T2 months of the 31 volunteers (n = 3), having applied the cream according to the invention to one side of the face and placebo contralateral, were performed.

**Table 20: Variation of the attenuation of the red light on the face of 31 volunteers**

| **Translucymeter^{®}** | **Cream according to the invention** | **Placebo** |
|---|---|---|
| **Arbitrary units** | | |
| **Mean +/- standard deviation** | 225.2 +/- 12.4 | 219.9 +/- 14.3 |
| **% variation vs. Placebo** | + 2.4% | |
| **Significance** | *p<0.01* | |
| **Maximum** | 11.8% | |
| **Responders**** | 71% | |

| | | |
|---|---|---|
| ** % of persons with a difference in favor of the side treated with the cream according to the invention. | | |

It can be seen that the attenuation value of the light is significantly greater by + 2.4% (*p <0.01*) on the side of the face which has received the cream according to the invention for two months compared with that which has received the placebo. These data indicate that the side treated with the cream according to the invention is denser than the other side.

### Facial pore supporting tissues by laser confocal microscopy

### Principe:

As explained above, the supporting tissues lose density with age, which results in the impression of having a skin with more open pores. At an intermediate time (T1 month) a study was carried out on persons who had clearly visible pores at T0 (n=23): horizontal optical sections of the skin were made using a confocal laser microscope (VivaScope^{®} 3000, Mavig GmbH).

This camera takes pictures at different depths of the skin and allows a completely painless and non-invasive way of "traveling" in the latter to the papillary dermis (near the epidermis). The optical sections thus produced are only 2.6 microns (µm) deep apart from each other.

The set of horizontal photos, taken at the level of the dermis of two pores, made it possible to extract the peripheral zone of the pores. An image of a vertical channel surrounded by its supporting tissue is obtained, this image allows the analysis and quantification of the density of the supporting tissue.

### Results:

Photographs, taken on volunteers, illustrate the effect of the cream according to the invention between T0 and T2months on the pore opening parameter. In these volunteers, there is a marked change in the appearance of the skin after application.

**Table 21: Variation of the intensity of the collagenous sheath after application of the cream according to the invention (n=2 measurements, 23 volunteers).**

| | **Cream according to the invention** | | **Placebo** | |
|---|---|---|---|---|
| | **T0** | **T4 weeks** | **T0** | **T4 weeks** |
| **Mean** | 57.8 | 66.1 | 62.8 | 64.3 |
| **Standard deviation** | 14.5 | 18.1 | 12.2 | 13.3 |
| % **variation vs. T0** | | +14.4% | | +2.4% |
| **Significance Maximum** | | *p<0.01* +50% | | *nsd* |
| **Significance vs. placebo** | *p<0.05* | | | |

Against placebo: parametric test, unilateral; *p<0. 09* bilaterally.

The results show that the application for only four weeks of the cream according to the invention makes it possible to improve the densification of the peripheral pore tissue by 14.4% in one month this very significantly (*p*<*0.05*) compared to placebo which does not significantly improve this tissue (+2.4%, *nsd*). These results confirm those obtained using the cutometer and the translucometer: it is demonstrated that the cream according to the invention re-pulps and re-densifies skin supporting tissues better and significantly compared to placebo. No significant difference between the two sides at T0 was observed.

### Evaluation of the texture by a Goniolux^{®}

### Principe:

With ageing, the surface of the skin loses its homogeneity because of the increase of the microroughness and the enlargement of the pores in particular. A young skin with a regular and uniform texture will better reflect the light it receives and will give the skin a satin finish. It is the loss of homogeneity of the texture that changes the way the light is reflected by it and can make the skin duller, less radiant. The cream according to the invention has a beneficial effect of resurfacing the skin in addition to its plumping effect of the dermis.

Quantification of the light scattering at the surface of the skin can be carried out 1) by clinical observation, which is a very dependent of the operator, 2) by the extraction of the gloss components on photos, a method closely related to the quality of photos capturing this brilliance, or 3) by metrological optical means of the goniometer type, a method which was retained here by using a Goniolux^{®} (Orion Technoloabtm, France).

Goniolux^{®} measures the intensity of light reflected from different angles. Its cylinder is applied in a controlled manner on the selected skin area. Its illumination system sends a light whose return is collected by means of several optical fibers regularly distributed on a hemisphere. The light reflected by the skin breaks down into specular reflected light (mirror effect) and diffuse reflected light (satin effect), a parameter that has been used. The scale of the device is in arbitrary units and then converted into percentage (hence from 0 to 100). On Caucasian skin, the dynamics evolve little: 35% for a very shiny skin to 45% for a very satiny skin. The variations were expressed using this scale.

### Results:

**Table 22: Variation of the satin aspect of the skin after application of the cream according to the invention or of its placebo (n = 3 measurements, 31 volunteers)**

| **Rate of scattered light (%)** | Cream **according** to the **invention** | | **Placebo** | |
|---|---|---|---|---|
| | **T0** | **T4 weeks** | **T0** | **T4 weeks** |
| **Mean** | 40.0 | 42.2 | 40.0 | 40.8 |
| **Standard deviation** | 0.4 | 0.8 | 0.3 | 0.6 |
| **Difference** | | 2.2 | | 0.8 |
| **Physiological variation** | | 22% | | 8% |
| **Significance** | | *p<0.01* | | *p<0.01* |
| **Maximum** | | +40% | | |
| **Responders** | | 100% | | |
| **Significance vs Placebo** | *p<0.01* | | | |

It is observed, after one month of application with the cream according to the invention, that the level of scattered light is increased significantly, on the physiological scale, by 22% (*p*<*0.01*) compared to the beginning of the test and compared to placebo. At the same time, the massage with the placebo also improves the satin aspect but more moderately (+8%).

This leads to a more satin-like face where light diffuses to the surface of the skin and produces a naturally softer illumination.

### Evaluation of skin texture of the cheek by print analysis

### Principe:

The variation of the texture was measured using another technique, robust and proven, which perfectly complements the method used previously and the observations on photos. At each of the times, a negative molding of the cheek skin of the volunteers was performed using a silicone polymer. An acquisition is then made by the technique of drop shadows (Visioline, Monaderm), then the analysis is conducted with a specialized software.

In order to reflect the texture of the micro-depressional network of the skin, the isotropy parameter was chosen. It is known that a young skin has a high surface homogeneity, being isotropic. This skin diffuses the light better because it has microgrooves well present and oriented in all directions, it also has fewer surface defects and they are less exacerbated. The footprints of 28 volunteers were retained.

### Results:

**Table 23: Variation of isotropy after application of the cream according to the invention or of its placebo on the cheeks. (n=28)**

| | **Cream of the invention** | | **Placebo** | |
|---|---|---|---|---|
| | **T0** | **T4 weeks** | **T0** | **T4 weeks** |
| **Mean** | 31.8 | 35.0 | 36.3 | 35.3 |
| **Standard deviation** | 7.1 | 7.1 | 7.5 | 6.9 |
| **% variation *vs*. T0** | | +10% | | -2.7% |
| **Significance** | | *p<0.05* | | *nsd* |
| **Maximum** | | +121% | | |
| **Responders** | | 71% | | |
| **Significance vs. placebo** | *p<0.06* | | | |

The data of Table 23 indicate that after only 1 month, the isotropy of the cheek skin is markedly and significantly increased by 10% after application of the cream according to the invention (*p <0.05 vs* T0 and *p<0.06* vs placebo). The application of the placebo, on the contrary, reduces this parameter but without significance (-2.7%, *nsd*)*.* These results obtained with Goniolux^{®} and photos demonstrate that the texture of the skin is improved. This is related in particular to the improvement of the qualities of the underlying skin (firmness, tone, elasticity), the reinforcement of the peripheral supporting tissues to the cutaneous pores and the energetic dynamism.

## Claims

1. Use of at least one cyclic peptide as an active component in a non-therapeutic cosmetic treatment of the skin and/or its appendages, said cyclic peptide consisting of at least five amino acids including at least one proline (Pro) and at least two phenylalanine (Phe), the other amino acids being selected from the group comprising leucine (Leu), isoleucine (Ile), valine (Val), alanine (Ala), glycine (Gly) Methionine (Met) and Tryptophan (Trp), Methionine (Met), when present, may be unoxidized or oxidized,
wherein said cyclic peptide comprises at most 15 amino acids, wherein the cyclic peptide comprises at least one leucine, and
wherein the cyclic peptide comprises at least two phenylalanines and/or at least two prolines.

2. Use according to claim 1, wherein the cyclic peptide comprises at least one Pro-Phe-Phe sequence.

3. Use according to anyone of claims 1-2, wherein the cyclic peptide comprises one Pro-Pro-Phe-Phe sequence.

4. Use according to anyone of claims 1-3, wherein the cyclic peptide comprises one Ile-Met-Leu or Val-Met-Leu sequence.

5. Use according to anyone of claims 1-4, wherein the cyclic peptide comprises two methionines.

6. Use according to anyone of claims 1-5, wherein the cyclic peptide comprises one tryptophan.

7. Use according to claim 6, wherein the cyclic peptide comprises the Pro-Phe-Phe-Trp sequence.

8. Use according to anyone of claims 1-7, wherein the cyclic peptide comprises at most twelve aminoacids.

9. Use according to claim 1, wherein said at least one cyclic peptide is chosen in the group comprising:
- the *Cyclo*(-Met-Leu-Val-Phe-Pro-Leu-Phe-Ile) (SEQ ID NO: 4);
- the *Cyclo*(-Met-Leu-Ile-Pro-Pro-Phe-Phe-Val-Ile) (SEQ ID NO: 5);
- the *Cyclo*(-Ile-Leu-Val-Pro-Pro-Phe-Phe-Leu-Ile) (SEQ ID NO: 6);
- the *Cyclo*(-Met-Leu-Met-Pro-Phe-Phe-Trp-Ile) (SEQ ID NO: 7);
- the *Cyclo*(-Met-Leu-Leu-Pro-Phe-Phe-Trp-Ile) (SEQ ID NO: 8); and
- the Cyclo(-Met-Leu-Met-Pro-Phe-Phe-Trp-Val) (SEQ ID NO: 9).

10. Use according to claim 9, wherein a mixture of at least two cyclic peptides is used, said at least two cyclic peptides being chosen in the group comprising:
- the Cyclo(-Met-Leu-Val-Phe-Pro-Leu-Phe-Ile) (SEQ ID NO: 4);
- the Cyclo(-Met-Leu-Ile-Pro-Pro-Phe-Phe-Val-Ile) (SEQ ID NO: 5); and
- the *Cyclo*(-Ile-Leu-Val-Pro-Pro-Phe-Phe-Leu-Ile) (SEQ ID NO: 6).

11. Use according to claim 10, wherein the mixture of cyclic peptides comprises the three following cyclic peptides:
- the *Cyclo*(-Met-Leu-Val-Phe-Pro-Leu-Phe-Ile) (SEQ ID NO: 4);
- the *Cyclo*(-Met-Leu-Ile-Pro-Pro-Phe-Phe-Val-Ile) (SEQ ID NO: 5); and
- the *Cyclo*(-Ile-Leu-Val-Pro-Pro-Phe-Phe-Leu-Ile) (SEQ ID NO: 6).

12. Use according to claim 9, wherein the mixture of cyclic peptides comprises:
- At least 50% in weight with regard to the total weight of the mixture of cyclic peptides of the following three cyclic peptides:
∘ the *Cyclo*(-Met-Leu-Val-Phe-Pro-Leu-Phe-Ile) (SEQ ID NO: 4);
∘ the *Cyclo*(-Met-Leu-Ile-Pro-Pro-Phe-Phe-Val-Ile) (SEQ ID NO: 5); and
∘ the *Cyclo*(-Ile-Leu-Val-Pro-Pro-Phe-Phe-Leu-Ile) (SEQ ID NO: 6);
- the balance in weight % with regard to the total weight of the mixture of cyclic peptides comprising at least one cyclic peptide chosen in the group comprising:
∘ the *Cyclo*(-Met-Leu-Met-Pro-Phe-Phe-Trp-Ile) (SEQ ID NO: 7);
∘ the *Cyclo*(-Met-Leu-Leu-Pro-Phe-Phe-Trp-Ile) (SEQ ID NO: 8); and
∘ the *Cyclo*(-Met-Leu-Met-Pro-Phe-Phe-Trp-Val) (SEQ ID NO: 9).

13. Use according to claim 12, wherein the mixture of cyclic peptides comprises:
- Approximately 15 to 25% in weight with regard to the total weight of said cyclic peptide mixture of *Cyclo*(-Met-Leu-Val-Phe-Pro-Leu-Phe-Ile) (SEQ ID NO: 4);
- Approximately 15 to 25% in weight with regard to the total weight of said cyclic peptide mixture of *Cyclo*(-Met-Leu-Ile-Pro-Pro-Phe-Phe-Val-Ile) (SEQ ID NO: 5);
- Approximately 15 to 25% in weight with regard to the total weight of said cyclic peptide mixture of *Cyclo*(-Ile-Leu-Val-Pro-Pro-Phe-Phe-Leu-Ile) (SEQ ID NO: 6); and
- The balance in weight % with regard to the total weight of cyclic peptide mixture being a mixture of the cyclic peptides *Cyclo*(-Met-Leu-Met-Pro-Phe-Phe-Trp-Ile) (SEQ ID NO: 7), *Cyclo*(-Met-Leu-Leu-Pro-Phe-Phe-Trp-Ile) (SEQ ID NO: 8) and *Cyclo*(-Met-Leu-Met-Pro-Phe-Phe-Trp-Val) (SEQ ID NO: 9).

14. Use according to anyone of claims 1-13, wherein the cyclic peptide(s) are extracted from flax seed oil.

15. Use according to anyone of claims 1-14, wherein said cosmetic treatment is for improving the properties of elasticity, firmness, texture and/or radiance of skin.

16. Use according to claim 15, wherein the treatmen tis chosen among a treatment of fines lines and wrinkles, a smoothing treatment, a firming treatment, a restructuring treatment, a treatment against sagging, a treatment for reducing the size of pores and/or for reducing the shine of skin due to an exces of sebum and/or for decreasing cutaneous micro-inflammations.

17. Cosmetic active ingredient comprising in a physiologically acceptable medium a mixture of cyclic peptides comprising:
- Approximately 15 to 25% in weight with regard to the total weight of said cyclic peptide mixture of *Cyclo*(-Met-Leu-Val-Phe-Pro-Leu-Phe-Ile) (SEQ ID NO: 4);
- Approximately 15 to 25% in weight with regard to the total weight of said cyclic peptide mixture of *Cyclo*(-Met-Leu-Ile-Pro-Pro-Phe-Phe-Val-Ile) (SEQ ID NO: 5);
- Approximately 15 to 25% in weight with regard to the total weight of said cyclic peptide mixture of *Cyclo*(-Ile-Leu-Val-Pro-Pro-Phe-Phe-Leu-Ile) (SEQ ID NO: 6); and
- The balance in weight % with regard to the total weight of cyclic peptide mixture being a mixture of the cyclic peptides *Cyclo*(-Met-Leu-Met-Pro-Phe-Phe-Trp-Ile) (SEQ ID NO: 7), *Cyclo*(-Met-Leu-Leu-Pro-Phe-Phe-Trp-Ile) (SEQ ID NO: 8) and *Cyclo*(-Met-Leu-Met-Pro-Phe-Phe-Trp-Val) (SEQ ID NO: 9).

18. Ingredient according to claim 17, wherein the cyclic peptide mixture is an extract of flax seed oil.

19. Ingredient according to claim 17 or claim 18, wherein said ingredient further comprises at least one cosmetic active adapted to act on the properties of the epidermis and/or the *stratum corneum* and/or on the decrease of the production of sebum by the sebocytes and/or having an astringent activity.

20. Ingredient according to claim 19, wherein said further active is at least one alkyl-phthalide chosen in the group comprising the sedanenolide, the sedanolide and the 3-n-butylphtalide or an extract of *Apium graveolens* seeds comprising a mixture of sedanenolide, sedanolide and 3-n-butylphtalide.

21. Cosmetic or topical therapeutical composition comprising an ingredient according to anyone of claims 17-20.

22. Non-therapeutic method for improving the aestethical appearance of skin comprising the topical application of an effective amount of the cosmetic composition of claim 21.

## Patentansprüche

1. Verwendung von mindestens einem zyklischen Peptid als Wirkkomponente in einer nicht therapeutischen kosmetische Behandlung der Haut und/oder ihrer Anhangsgebilde, wobei das zyklische Peptid aus mindestens fünf Aminosäuren besteht, die mindestens ein Prolin (Pro) und mindestens zwei Phenylalanine (Phe) beinhalten, wobei die anderen Aminosäuren ausgewählt sind aus der Gruppe umfassend Leucin (Leu), Isoleucin (Ile), Valin (Val), Alanin (Ala), Glycin (Gly), Methionin (Met) und Tryptophan (Trp), wobei Methionin (Met), sofern es vorliegt, nicht oxidiert oder oxidiert sein kann,
wobei das zyklische Peptid höchstens 15 Aminosäuren umfasst,
wobei das zyklische Peptid mindestens ein Leucin umfasst, und
wobei das zyklische Peptid mindestens zwei Phenylalanine und/oder mindestens zwei Proline umfasst.

2. Verwendung nach Anspruch 1, wobei das zyklische Peptid mindestens eine Pro-Phe-Phe-Sequenz umfasst.

3. Verwendung nach einem der Ansprüche 1-2, wobei das zyklische Peptid eine Pro-Pro-Phe-Phe-Sequenz umfasst.

4. Verwendung nach einem der Ansprüche 1-3, wobei das zyklische Peptid eine Ile-Met-Leu- oder Val-Met-Leu-Sequenz umfasst.

5. Verwendung nach einem der Ansprüche 1-4, wobei das zyklische Peptid zwei Methionine umfasst.

6. Verwendung nach einem der Ansprüche 1-5, wobei das zyklische Peptid ein Tryptophan umfasst.

7. Verwendung nach Anspruch 6, wobei das zyklische Peptid die Pro-Phe-Phe-Trp-Sequenz umfasst.

8. Verwendung nach einem der Ansprüche 1-7, wobei das zyklische Peptid höchstens zwölf Aminosäuren umfasst.

9. Verwendung nach Anspruch 1, wobei das mindestens eine zyklische Peptid ausgewählt ist aus der Gruppe umfassend:
- das Cyclo(-Met-Leu-Val-Phe-Pro-Leu-Phe-Ile) (SEQ ID NO: 4);
- das Cyclo(-Met-Leu-Ile-Pro-Pro-Phe-Phe-Val-Ile) (SEQ ID NO: 5);
- das Cyclo(-Ile-Leu-Val-Pro-Pro-Phe-Phe-Leu-Ile) (SEQ ID NO: 6);
- das Cyclo(-Met-Leu-Met-Pro-Phe-Phe-Trp-Ile) (SEQ ID NO: 7);
- das Cyclo(-Met-Leu-Leu-Pro-Phe-Phe-Trp-Ile) (SEQ ID NO: 8); und
- das Cyclo(-Met-Leu-Met-Pro-Phe-Phe-Trp-Val) (SEQ ID NO: 9).

10. Verwendung nach Anspruch 9, wobei eine Mischung von mindestens zwei zyklischen Peptiden verwendet wird, wobei die mindestens zwei zyklischen Peptide ausgewählt sind aus der Gruppe umfassend:
- das Cyclo(-Met-Leu-Val-Phe-Pro-Leu-Phe-Ile) (SEQ ID NO: 4);
- das Cyclo(-Met-Leu-Ile-Pro-Pro-Phe-Phe-Val-Ile) (SEQ ID NO: 5); und
- das Cyclo(-Ile-Leu-Val-Pro-Pro-Phe-Phe-Leu-Ile) (SEQ ID NO: 6).

11. Verwendung nach Anspruch 10, wobei die Mischung von zyklischen Peptiden die drei folgenden zyklischen Peptide umfasst:
- das Cyclo(-Met-Leu-Val-Phe-Pro-Leu-Phe-Ile) (SEQ ID NO: 4);
- das Cyclo(-Met-Leu-Ile-Pro-Pro-Phe-Phe-Val-Ile) (SEQ ID NO: 5); und
- das Cyclo(-Ile-Leu-Val-Pro-Pro-Phe-Phe-Leu-Ile) (SEQ ID NO: 6).

12. Verwendung nach Anspruch 9, wobei die Mischung von zyklischen Peptiden Folgendes umfasst:
- Mindestens 50 Gew.-% bezogen auf das Gesamtgewicht der Mischung von zyklischen Peptiden der folgenden drei zyklischen Peptide:
∘ das Cyclo(-Met-Leu-Val-Phe-Pro-Leu-Phe-Ile) (SEQ ID NO: 4);
∘ das Cyclo(-Met-Leu-Ile-Pro-Pro-Phe-Phe-Val-Ile) (SEQ ID NO: 5); und
∘ das Cyclo(-Ile-Leu-Val-Pro-Pro-Phe-Phe-Leu-Ile) (SEQ ID NO: 6);
- den Rest der Gew.-% bezogen auf das Gesamtgewicht der Mischung von zyklischen Peptiden, der mindestens ein zyklisches Peptid umfasst, das ausgewählt ist aus der Gruppe umfassend:
∘ das Cyclo(-Met-Leu-Met-Pro-Phe-Phe-Trp-Ile) (SEQ ID NO: 7);
∘ das Cyclo(-Met-Leu-Leu-Pro-Phe-Phe-Trp-Ile) (SEQ ID NO: 8); und
∘ das Cyclo(-Met-Leu-Met-Pro-Phe-Phe-Trp-Val) (SEQ ID NO: 9).

13. Verwendung nach Anspruch 12, wobei die Mischung von zyklischen Peptiden Folgendes umfasst:
- Etwa 15 bis 25 Gew.-% bezogen auf das Gesamtgewicht der zyklischen Peptidmischung Cyclo(-Met-Leu-Val-Phe-Pro-Leu-Phe-Ile) (SEQ ID NO: 4);
- Etwa 15 bis 25 Gew.-% bezogen auf das Gesamtgewicht der zyklischen Peptidmischung Cyclo(-Met-Leu-Ile-Pro-Pro-Phe-Phe-Val-Ile) (SEQ ID NO: 5);
- Etwa 15 bis 25 Gew.-% bezogen auf das Gesamtgewicht der zyklischen Peptidmischung Cyclo(-Ile-Leu-Val-Pro-Pro-Phe-Phe-Leu-Ile) (SEQ ID NO: 6); und
- Den Rest der Gew.-% bezogen auf das Gesamtgewicht einer zyklischen Peptidmischung, der eine Mischung der zyklischen Peptide Cyclo(-Met-Leu-Met-Pro-Phe-Phe-Trp-Ile) (SEQ ID NO: 7), Cyclo(-Met-Leu-Leu-Pro-Phe-Phe-Trp-Ile) (SEQ ID NO: 8) und Cyclo(-Met-Leu-Met-Pro-Phe-Phe-Trp-Val) (SEQ ID NO: 9) ist.

14. Verwendung nach einem der Ansprüche 1-13, wobei das/die zyklische(n) Peptid(e) aus Leinöl extrahiert ist/sind.

15. Verwendung nach einem der Ansprüche 1-14, wobei die kosmetische Behandlung der Verbesserung der Eigenschaften Elastizität, Festigkeit, Textur und/oder Strahlkraft der Haut dient.

16. Verwendung nach Anspruch 15, wobei die Behandlung ausgewählt ist aus einer Behandlung feiner Linien und Falten, einer Glättungsbehandlung, einer Festigungsbehandlung, einer Restrukturierungsbehandlung, einer Behandlung gegen Schlaffheit, einer Behandlung zur Reduzierung der Porengröße und/oder zur Reduzierung glänzender Haut aufgrund von überschüssigem Talg und/oder zur Verminderung von Mikroentzündungen der Haut.

17. Kosmetischer Wirkstoff, umfassend, in einem physiologisch akzeptablen Medium, eine Mischung zyklischer Peptide, umfassend:
- Etwa 15 bis 25 Gew.-% bezogen auf das Gesamtgewicht der zyklischen Peptidmischung Cyclo(-Met-Leu-Val-Phe-Pro-Leu-Phe-Ile) (SEQ ID NO: 4);
- Etwa 15 bis 25 Gew.-% bezogen auf das Gesamtgewicht der zyklischen Peptidmischung Cyclo(-Met-Leu-Ile-Pro-Pro-Phe-Phe-Val-Ile) (SEQ ID NO: 5);
- Etwa 15 bis 25 Gew.-% bezogen auf das Gesamtgewicht der zyklischen Peptidmischung Cyclo(-Ile-Leu-Val-Pro-Pro-Phe-Phe-Leu-Ile) (SEQ ID NO: 6); und
- Den Rest der Gew.-% bezogen auf das Gesamtgewicht einer zyklischen Peptidmischung, der eine Mischung der zyklischen Peptide Cyclo(-Met-Leu-Met-Pro-Phe-Phe-Trp-Ile) (SEQ ID NO: 7), Cyclo(-Met-Leu-Leu-Pro-Phe-Phe-Trp-Ile) (SEQ ID NO: 8) und Cyclo(-Met-Leu-Met-Pro-Phe-Phe-Trp-Val) (SEQ ID NO: 9) ist.

18. Inhaltsstoff nach Anspruch 17, wobei die zyklische Peptidmischung ein Extrakt von Leinöl ist.

19. Inhaltsstoff nach Anspruch 17 oder Anspruch 18, wobei der Inhaltsstoff ferner mindestens einen kosmetischen Wirkstoff umfasst, der dazu geeignet ist, auf die Eigenschaften der Epidermis und/oder des Stratum corneum und/oder in Bezug auf die Verminderung der Produktion von Talg durch die Sebozyten einzuwirken und/oder antibakterielle Wirkung aufweist.

20. Inhaltsstoff nach Anspruch 19, wobei der weitere Wirkstoff mindestens ein Alkyl-Phthalid ist, das ausgewählt ist aus der Gruppe umfassend das Sedanenolid, das Sedanolid und das 3-n-Butylphtalid oder ein Extrakt aus Samen von Apium graveolens, die eine Mischung aus Sedanenolid, Sedanolid und 3-n-Butylphtalid umfassen.

21. Kosmetische oder topische therapeutische Zusammensetzung, umfassend einen Inhaltsstoff nach einem der Ansprüche 17-20.

22. Nicht therapeutisches Verfahren zum Verbessern des ästhetischen Erscheinungsbildes der Haut, umfassend die topische Anwendung einer wirksamen Menge der kosmetischen Zusammensetzung von Anspruch 21.

## Revendications

1. Utilisation d'au moins un peptide cyclique comme composant actif dans un traitement cosmétique non-thérapeutique de la peau et/ou de ses annexes, ledit peptide cyclique étant constitué d'au moins cinq acides aminés incluant au moins une proline (Pro) et au moins deux phénylalanine (Phe), les autres acides aminés étant choisis dans le groupe comprenant la leucine (Leu), l'isoleucine (Ile), la valine (Val), l'alanine (Ala), la glycine (Gly), la méthionine (Met) et le tryptophane (Trp), la méthionine (Met), lorsqu'elle est présente, pouvant être non-oxydée ou oxydée,
dans laquelle le peptide cyclique comprend au plus 15 acides aminés,
dans laquelle ledit peptide cyclique comprend au moins une leucine, et
dans laquelle ledit peptide cyclique comprend au moins deux phénylalanines et/ou au moins deux prolines.

2. Utilisation selon la revendication 1, dans laquelle le peptide cyclique comprend au moins une séquence Pro-Phe-Phe.

3. Utilisation selon l'une quelconque des revendications 1-2, dans laquelle le peptide cyclique comprend une séquence Pro-Pro-Phe-Phe.

4. Utilisation selon l'une quelconque des revendications 1-3, dans laquelle le peptide cyclique comprend une séquence Ile-Met-Leu ou Val-Met-Leu.

5. Utilisation selon l'une quelconque des revendications 1-4, dans laquelle le peptide cyclique comprend deux méthionines.

6. Utilisation selon l'une quelconque des revendications 1-5, dans laquelle le peptide cyclique comprend un tryptophane.

7. Utilisation selon la revendication 6, dans laquelle le peptide cyclique comprend la séquence Pro-Phe-Phe-Trp.

8. Utilisation selon l'une quelconque des revendications 1-7, dans laquelle le peptide cyclique comprend au plus douze acides aminés.

9. Utilisation selon la revendication 1, dans laquelle ledit au moins un peptide cyclique est choisi dans le groupe comprenant :
- le *Cyclo*(-Met-Leu-Val-Phe-Pro-Leu-Phe-Ile) (SEQ ID NO: 4) ;
- le *Cyclo*(-Met-Leu-Ile-Pro-Pro-Phe-Phe-Val-Ile) (SEQ ID NO: 5) ;
- le *Cyclo*(-Ile-Leu-Val-Pro-Pro-Phe-Phe-Leu-Ile) (SEQ ID NO: 6) ;
- le *Cyclo*(-Met-Leu-Met-Pro-Phe-Phe-Trp-Ile) (SEQ ID NO: 7) ;
- le *Cyclo*(-Met-Leu-Leu-Pro-Phe-Phe-Trp-Ile) (SEQ ID NO: 8) ; et
- le *Cyclo*(-Met-Leu-Met-Pro-Phe-Phe-Trp-Val) (SEQ ID NO: 9).

10. Utilisation selon la revendication 9, dans laquelle un mélange d'au moins deux peptides cycliques est utilisé, lesdits au moins deux peptides cycliques étant choisis dans le groupe comprenant :
- le *Cyclo*(-Met-Leu-Val-Phe-Pro-Leu-Phe-Ile) (SEQ ID NO: 4) ;
- le *Cyclo*(-Met-Leu-Ile-Pro-Pro-Phe-Phe-Val-Ile) (SEQ ID NO: 5) ; et
- le *Cyclo*(-Ile-Leu-Val-Pro-Pro-Phe-Phe-Leu-Ile) (SEQ ID NO: 6).

11. Utilisation selon la revendication 10, dans laquelle le mélange de peptides cycliques comprend les trois peptides cycliques suivants :
- le *Cyclo*(-Met-Leu-Val-Phe-Pro-Leu-Phe-Ile) (SEQ ID NO: 4) ;
- le *Cyclo*(-Met-Leu-Ile-Pro-Pro-Phe-Phe-Val-Ile) (SEQ ID NO: 5) ; et
- le *Cyclo*(-Ile-Leu-Val-Pro-Pro-Phe-Phe-Leu-Ile) (SEQ ID NO: 6) ;

12. Utilisation selon la revendication 9, dans laquelle un mélange de peptides cycliques comprend:
- au moins 50% en % en poids par rapport au poids total dudit mélange de peptides cycliques des trois peptides cycliques suivants :
∘ le Cyclo(-Met-Leu-Val-Phe-Pro-Leu-Phe-Ile) (SEQ ID NO: 4) ;
∘ le Cyclo(-Met-Leu-Ile-Pro-Pro-Phe-Phe-Val-Ile) (SEQ ID NO: 5) ; et
∘ le Cyclo(-Ile-Leu-Val-Pro-Pro-Phe-Phe-Leu-Ile) (SEQ ID NO: 6) ;
- le complément en % en poids par rapport au poids total dudit mélange de peptides cycliques comprenant au moins un peptide cyclique choisi dans le groupe comprenant :
∘ le *Cyclo*(-Met-Leu-Met-Pro-Phe-Phe-Trp-Ile) (SEQ ID NO: 7) ;
∘ le *Cyclo*(-Met-Leu-Leu-Pro-Phe-Phe-Trp-Ile) (SEQ ID NO: 8) ; et
∘ le *Cyclo*(-Met-Leu-Met-Pro-Phe-Phe-Trp-Val) (SEQ ID NO: 9).

13. Utilisation selon la revendication 12, dans laquelle le mélange de peptides cycliques comprend :
- environ 15 à 25% en poids par rapport au poids total dudit mélange de peptides cycliques de *Cyclo*(-Met-Leu-Val-Phe-Pro-Leu-Phe-Ile) (SEQ ID NO: 4) ;
- environ 15 à 25% en poids par rapport au poids total dudit mélange de peptides cycliques de *Cyclo*(-Met-Leu-Ile-Pro-Pro-Phe-Phe-Val-Ile) (SEQ ID NO: 5) ;
- environ 15 à 25% en poids par rapport au poids total dudit mélange de peptides cycliques de *Cyclo*(-Ile-Leu-Val-Pro-Pro-Phe-Phe-Leu-Ile) (SEQ ID NO: 6) ; et
- le complément en % en poids par rapport au poids total de mélange de peptides cycliques étant un mélange des peptides cycliques Cyclo(-Met-Leu-Met-Pro-Phe-Phe-Trp-Ile) (SEQ ID NO: 7), *Cyclo*(-Met-Leu-Leu-Pro-Phe-Phe-Trp-Ile) (SEQ ID NO: 8) et *Cyclo*(-Met-Leu-Met-Pro-Phe-Phe-Trp-Val) (SEQ ID NO: 9).

14. Utilisation selon l'une quelconque des revendications 1-13, dans laquelle le(s) peptide(s) cyclique(s) sont extraits d'huile de graines de lin.

15. Utilisation selon l'une quelconque des revendications 1-14, dans laquelle ledit traitement cosmétique est destiné à améliorer les propriétés élastiques, la fermeté, la texture et/ou l'éclat de la peau.

16. Utilisation selon la revendication 15, dans laquelle le traitement est choisi parmi un traitement des rides et des ridules, un traitement lissant, raffermissant, restructurant, contre l'affaissement, pour réduire la taille des pores et/ou pour réduire la brillance de la peau due à un excès de sébum et/ou pour diminuer des micro-inflammations cutanées.

17. Ingrédient actif cosmétique comprenant dans un milieu physiologiquement acceptable un mélange de peptides cycliques comprenant :
- environ 15 à 25% en poids par rapport au poids total dudit mélange de peptides cycliques de Cyclo(-Met-Leu-Val-Phe-Pro-Leu-Phe-Ile) (SEQ ID NO: 4) ;
- environ 15 à 25% en poids par rapport au poids total dudit mélange de peptides cycliques de Cyclo(-Met-Leu-Ile-Pro-Pro-Phe-Phe-Val-Ile) (SEQ ID NO: 5) ;
- environ 15 à 25% en poids par rapport au poids total dudit mélange de peptides cycliques de Cyclo(-Ile-Leu-Val-Pro-Pro-Phe-Phe-Leu-Ile) (SEQ ID NO: 6) ; et
- le complément en % en poids par rapport au poids dudit mélange de peptides cycliques étant un mélange des peptides cycliques *Cyclo*(-Met-Leu-Met-Pro-Phe-Phe-Trp-Ile) (SEQ ID NO: 7), *Cyclo*(-Met-Leu-Leu-Pro-Phe-Phe-Trp-Ile) (SEQ ID NO: 8) et *Cyclo*(-Met-Leu-Met-Pro-Phe-Phe-Trp-Val) (SEQ ID NO: 9).

18. Ingrédient selon la revendication 17, dans lequel le mélange de peptides cycliques est un extrait d'huile de graines de lin.

19. Ingrédient selon la revendication 17 ou la revendication 18, dans lequel ledit ingrédient comprend en outre au moins un actif cosmétique adapté à agir sur les propriétés de l'épiderme et/ou du *stratum corneum* et/ou sur la baisse de la production de sébum par les sébocytes et/ou ayant une activité astringeante.

20. Ingrédient selon la revendication 19, dans lequel ledit autre ingrédient actif est au moins un alkyl-phthalide choisi dans le groupe comprenant le sédanénolide, le sédanolide et le 3-n-butylphtalide ou un extrait de graines d'*Apium graveolens* comprenant un mélange de sédanenolide, sédanolide et de 3-n-butylphtalide.

21. Composition cosmétique ou dermatologique comprenant un ingrédient selon l'une quelconque des revendications 17-20.

22. Procédé non-thérapeutique pour améliorer l'apparence esthétique de la peau comprenant l'application topique sur la peau d'une quantité efficace d'une composition cosmétique selon la revendication 21.
